(19) Europäisches Patentamt
European Patent Office
Office européen des brevets

(11) **EP 4 555 924 A1**

(12) **EUROPEAN PATENT APPLICATION**

(43) Date of publication:
**21.05.2025 Bulletin 2025/21**

(21) Application number: **23306981.4**

(22) Date of filing: **15.11.2023**

(51) International Patent Classification (IPC):
*A61B 5/021* (2006.01)   *A61B 5/024* (2006.01)
*A61B 5/374* (2021.01)   *G16H 20/10* (2018.01)
*G16H 50/20* (2018.01)   *G16H 50/30* (2018.01)
*A61B 5/20* (2006.01)

(52) Cooperative Patent Classification (CPC):
**A61B 5/374; A61B 5/021; A61B 5/024;
A61B 5/4088; A61B 5/4821; A61B 5/7267;
A61B 5/7275; G16H 20/10; G16H 50/20;
G16H 50/30; G16H 50/70;** A61B 5/0022;
A61B 5/02405; A61B 5/201; A61B 5/4244;   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Assistance Publique - Hôpitaux de Paris
75012 Paris (FR)**

• **Institut National de Recherche en Informatique et
en Automatique
78150 Le Chesnay-Rocquencourt (FR)**

(72) Inventor: **The designation of the inventor has not
yet been filed**

(74) Representative: **Flesselles, Bruno F.G.
BF IP
36 rue Jean de la Fontaine
75016 Paris (FR)**

(54) **METHOD FOR DETERMINING FRAILTY USING ANESTHESIA DATA**

(57)   The invention is in the field of diagnosis and prognosis methods to detect the risk of frailty (risk of occurrence of an adverse health condition in a patient, including death), by combining data and markers developed using data obtained during anesthesia of the patient.

Figure 2

EP 4 555 924 A1

(52) Cooperative Patent Classification (CPC): (Cont.)
A61B 2503/08; A61B 2505/05; G16H 20/17;
G16H 20/40; G16H 70/20; G16H 70/40

**Description**

The invention is in the field of diagnosis and prognosis methods to detect the risk of frailty (risk of occurrence of an adverse health condition in a patient, including death), by combining data and markers developed using data obtained during anesthesia of the patient.

The rise of neurodegenerative and cardiovascular disorders poses a grave challenge to global healthcare, contributing significantly to mortality and straining resources. Early detection and intervention are crucial, yet current diagnostic strategies may overlook valuable tools. Among these, general anesthesia (used in over 300 million surgical procedures worldwide each year) holds powerful potential. The invention was developed on the basis that the assumption that general anesthesia (GA) may serve to identify physiological stress factors, associated with brain and cardiovascular health. By exploring individual patient responses under anesthesia, the aim is to tap into intraoperative data previously ignored, building early predictive markers for these chronic diseases.

In 2012, Sessler et al. (Anesthesiology. 2012 Jun;116(6):1195-203) conducted pioneering work exploring the impact of 'triple-low' (low mean arterial pressure (MAP) and low Bispectral Index (BIS) during a low minimum alveolar concentration (MAC) fraction; mean arterial pressure less than 75 mmHg, Bispectral Index less than 45, and minimum alveolar fraction less than 0.8) conditions, during GA, on postoperative mortality.

Despite significant findings, later research in 2019 (Sessler et al., Anesthesiology. 2019 Jan;130(1):72-82) suggested that real-time intervention based on these conditions did not lead to a reduction in 90-day mortality, which suggest that there is no strong relationship between responses to triple-low events and mortality.

The foundation of the present invention is rooted in a novel hypothesis that shows that anesthesia can be used in methods for frailty detection and occurrence of adverse effects (disease prediction). GA is considered as being a physiological stress test that reveals and provides insightful information about the brain and cardiovascular health.

By identifying specific markers obtained during GA procedure, the inventors were able to predict the development of cardiovascular disease, cognitive decline or death. Unique to the invention is the capability to predict long-term (2.5 years) prognosis. This extended outlook makes it possible to scrutinize the systemic health of patients, discerning between frailty and robustness while eliminating confounding influences of subsequent anesthesia or surgical procedures. It is also important to underline that the methods herein disclosed can essentially only be practically conducted within the confines of general anesthesia, as they need use of drugs used that are unsuitable for routine screening tests.

By examining intraoperative parameters, the inventors were able to create various scoring system for long-term poor prognosis such as neurocognitive decline, cardiovascular events, and mortality at > 2 years (2.5 years). This approach facilitates the detection of systemic frailty that might otherwise go unnoticed.

However, the methods herein disclosed could also be used to identify and develop other tests for prognosis for longer times. Indeed, the present teachings show use of intraoperative parameters (markers that were obtained during, or associated with anesthesia) in the development of methods to predict frailty of a subject.

In the context of the present invention, a "marker" is a data or an information obtained from or associated with a subject. It can be actual information about the subject (age, cardiovascular history), measurement of subject's physiological paramaters (EEG, ECG, arterial pressure...) or information about pharmacological elements that are administered to the subject (amount of anesthetic drug, of vasopressor drug...). Markers cannot always be used as such in the methods herein disclosed and may be processed to obtain a numerical value therefrom, that will be used in the methods. Such processes may include filtering, transformation, integration of a signal (analysis of EEG data), comparison with reference data (for hemodynamic data), normalization of the data (anesthetic data), modification of the data for taking into account the specific circumstances in which it was obtained (subject's weight, duration of surgery for vasopressor data), assigning a numerical value (score) to the data potentially already transformed (such as a binary value 0/1 for cardiovascular history, [0-3] score for other data).

Frailty is a medical condition of reduced function and health in older individuals. It is most often defined by physiological decline, with marked vulnerability to adverse health outcomes. Frail persons are more likely to be hospitalized, need long term care or die.

As indicated, the present invention intends to provide some markers that makes it possible to detect people with frailty, or having an increased frailty risk. The invention is based on the combination of readily measurable markers that can be obtained during anesthesia of a subject, as well as on the age. It is to be noted that the adverse heath disorder of patient is not an adverse effect due to anesthesia, but that the markers and data obtained during anesthesia reveal the frailty of the patient

In the context of the present application, systemic (or general) frailty indicates that the patient has an increased risk of a cardiovascular adverse event (myocardial infraction or cerebral stroke), of a neurodegenerative adverse event (cognitive decline) and/or of death within 2.5 years (post anesthesia), as compared with patients with no systemic frailty. One can also define cardiovascular frailty as a subgroup of systemic frailty (increased risk of a cardiovascular adverse event within 2.5 years post anesthesia) or neurologic frailty (increased risk of a neurodegenerative adverse event within 2.5 years post anesthesia) as subgroups of systemic frailty. Frailty thus includes systemic, cardiovascular and neurologic frailties.

The frailty risk that can be detected according to the methods herein disclosed is increased as compared with the general risk of a population of patients not having frailty. As an illustration, if the risk of the population of patients not having a given frailty is x %, the risk of patients with the given frailty would be higher than x %. A risk of x % within a population indicates that, in the population, x % of the patients present the required phenotype (here the given frailty). For the population patients defined with systemic frailty according to the methods herein disclosed, more than x% of the population present the phenotype (here the given frailty). Consequently, it is said that patients have an increased risk of having the phenotype.

It is reminded that the patients contemplated herein are the ones that have been subject to a general anesthesia, and the frailty is thus measured in these patients, some of whom being frail while others are not. Anesthesia, and the markers that are obtained from this procedure, thus makes it possible to reveal the frailty status of the patients.

The quality of a test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be understood as a curve allowing prediction of the quality of a test. It is best for the AUROC to be as closed as 1 as possible, this value describing a test which is 100 % specific and sensitive. Consequently, a test with a higher AUROC than another has a "better quality" than the other.

It is reminded that

(1) sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).

(2) specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).

(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).

(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

Generally, a diagnosis method comprises

i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

As a matter of illustration

i. the information that can be gathered from the patient can be gathered directly from the patient (here, information from the brain activity (electroencephalogram, EEG), or from the blood pressure), or indirectly from the patient (one can include here the parameters of the anesthesia)
ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease (or for patients who have been known to later evolve to a specific disease stage, for prognosis methods). Thresholds may exist, where a given percentage of patients having passed the threshold have the disease (such percentage being the PPV) and a percentage of the patients not having passed the threshold do not have the disease (such percentage being the NPV). For diseases where multiple clinical stages can be determined, such thresholds can discriminate the different stages. In this step ii, one may compare various types of information to their respective standards, in order to be able to reach a diagnostic in step iii (conclusion or deduction of the actual risk of the patient).

iii. the last step is actually making the diagnosis (resp. determining a prognosis) *i.e.* deciding whether or not the patient has the condition sought resp. whether or not the patient will evolve to a given clinical state), taking, in particular, into account the information gathered from the patient, the thresholds as described above. The physician may also take into account other elements (such as the consistency of the information gathered or the like) to make the diagnostic.

Some methods, such as the ones disclosed in the present application, shall also include a step i.a), which comprise the steps of modifying the information obtained in i) in order to obtain a new type of information, which is the one that is then compared to the standards in step ii. Such modification is the transformation of signal to numerical variables, and combination of the values of variables in a function (such as a logistic regression function or a Cox function), and obtaining an end value.

It is further to be noted that the transformation of the values obtained from a patient and the combination thereof in an algorithm or function as herein disclosed is part of a method and provides an intermediate result (an end value or index) that would then to be compared to a reference index (threshold), in order to be able to pose the diagnostic, even though the end value is informative by itself.

It is also to be noted that the tests herein disclosed are not "gold-standard" tests, in the sense that the output (end value or index calculated by the formulas herein disclosed) is not a definitive answer as to the state of the patient. Indeed, these tests are based on statistics and there may thus be false-positive or false-negative results, which is the reason why the specific experience of the physician in interpreting the index is of importance for making the prognosis and deciding which kind of follow up is to be made to be made for each patient.

Consequently, step iii as disclosed above is not direct and immediate from step ii, and the physician must interpret the result from the clinical and general context to be able to reach a conclusion, even though the predictive positive value and the negative predictive value are very high (in view of the high specificity and sensitivity of the tests), indicating that the relative risk is quite increased in the group of patients that respond positively to the tests as compared to the patients that do not respond positively. The tests are of great interest in provided a help to the physician when investigating a clinical case.

In order to identify patients with systemic frailty, cardiovascular frailty or neurologic frailty, the inventors used some markers obtained or generated from data gathered during anesthesia of the subject. These markers are thus used in *ex vivo* methods, generally after anesthesia of the subject (although it is conceivable that the diagnosis / prognosis test could be performed during anesthesia of the subject). It is to be noted that the tests are not practiced on the human body, in the sense that the diagnosis / prognosis can be reached without the need of the human body of the subject to be present. Indeed, these tests only need data that have been gathered, and are thus no different from the majority of diagnosis tests, that use sample gathered from the patient, without the need of the patient body to be present. Furthermore, the methods herein disclosed are computer-implemented, and thus technical method steps are performed by a computer device. As indicated above, some methods herein disclosed also comprise only the steps of generating an end value, which is an intermediate finding of diagnostic relevance, while other methods may also comprise the step of comparing the end value with predetermined values.

The inventors were able to demonstrate that the acquisition of data from at least one physiological marker associated with the cerebral state of the subject, and at least one physiological marker associated with the cardiovascular state of the subject, measured during anesthesia of the subject can be combined in a function, and that the end result thus obtained can be used to identify a frailty of the subject.

In other embodiments, it is advisable to use further information, related to the anesthesia regimen and conditions used to put the patient to sleep and to maintain the patient under stable anesthesia (quantity of anesthetic drug and/or of antalgic (painkiller) or analgesic used, notably morphine) and to combine sch information in a function together with the above data (cardiovascular and cerebral information).

In some embodiments, the age of the patient can also be used in the function that leads to the end result.

The invention thus relates to the use of physiological and optionally of pharmacological markers and/or of the age of a patient for determining whether a subject has an increased risk (or an absolute) to have an adverse effect (myocardial infraction, stroke, cognitive decline, death) or does not have this risk.

The invention thus relates to a method for determining whether a subject (especially a human subject) has an increased risk of having frailty, comprising

a) Providing data from at least one physiological marker associated with the cerebral state of the subject, and one physiological marker associated with the cardiovascular state of the subject, wherein these data have been obtained during anesthesia of the subject

b) Optionally providing data from at least one marker (parameter) associated with the anesthesia regimen (a marker associated with the product administered to the subject during anesthesia)

c) Optionally providing the age of the patient

d) Calculating numerical values from the data obtained in a) and in b) when provided

e) Combining the values, optionally normalized, calculated in d) in order to obtain an end value.

The end value is indicative of the presence or of the absence of an increased risk of having a frailty, and can be compared to reference values (cutoffs) to conclude on the actual risk. One interest of the method is that it is capable of predicting long-term events, while avoiding positive results in case of post-operative complications, when the function according to which the values are combined has been made as indicated below by excluding patients with post-operative complications (complications within 90 days).

In some embodiments, one can also use, in b), markers pertaining to the general state of the subject prior to anesthesia. In particular, one can use, as a marker, the simplified Lee's score (which is designed to predict the risk of major cardiac complications due to the surgery necessitating anesthesia, such as heart attacks or cardiac death, in patients undergoing non-cardiac surgery), or cardiovascular history of the patient (presence of cardiac events prior to anesthesia (history of any heart-related incidents such as myocardial infarction, heart failure, angina, or cardiac arrest) or of chronic conditions (arrhythmias or chronic heart failure, Arteriopathy of the Lower Limbs or Chronic Kidney Disease)). The value of these markers used would be the result of the Lee's score, and 0/1 in case of absence/presence of cardiovascular history. It is also possible to provide a score for these markers, as explained below. It is to be noted that these markers provide information as to the instant operative risk (the risk of anesthetizing the patient), and as to the post-operative risk (within 90 days), but not of an increased long time frailty risk (as compared to patients with the same characteristics).

In some embodiments, the method makes it possible to identify an increased risk of systemic frailty. In other embodiments, the method makes it possible to identify an increased risk of cardiovascular frailty. In other embodiments, the method makes it possible to identify an increased risk of neurologic frailty.

The method both makes it possible to identify the increased risk as indicated above (i.e. a risk that is higher in the population of subjects identified as being at risk by the method as compared to the population of subjects not identified at risk by the method). However, since the PPV and NPV of the tests are high, the methods also make it possible to identify patients with an absolute frailty risk (i.e. patients that can be predicted to have an adverse event (as described above) within 2.5 years after anesthesia).

It is reminded that the adverse events are either occurrence of myocardial infraction or cerebral stroke (cardiovascular adverse event), cognitive decline (neurodegenerative adverse event) and/or of death within 2.5 years (post anesthesia).

As indicated above, the methods are performed on data that has been harvested during anesthesia of a subject. The methods are however performed independently of the anesthesia. They can thus be performed after the anesthesia (once all needed data is available), but it is not unconceivable to perform the methods when the patient is still under general anesthesia, at a time "t", using data that has been obtained before the time "t". Indeed, the methods do not use instant data obtained during anesthesia.

**Physiological markers**

*Cerebral state of the subject*

A promising area of exploration is the application of EEG. As an affordable and established tool for GA monitoring, the EEG can yield insightful features, such as frequency band power or burst suppression rate. Using a physiological marker extracted from a EEG signal obtained during brain monitoring has been demonstrated to provide some information as to cerebral fragility.

As markers of cerebral activity, one can notably use the alpha power, associated with the brain alpha waves, and/or indexes that are routinely used during general anesthesia, and which provide insight into the direct and patient-specific effects of anesthesia on the brain, such as the bispectral Index (BIS, with devices developed and commercialized by Medtronic (Boulder, Colorado, USA), as described in Sigl and Chamoun (J Clin Monit. 1994 Nov;10(6):392-404) and Bowdle (Anesthesiol Clin. 2006 Dec;24(4):793-822). Using raw EEG data, an algorithm analyzes interprets the data, and display an end value ranging from 0 to 100, where a value of 0 represents absence of brain activity, and a value of 100 represents the awake state. It is generally sought to maintain BIS values between 40 to 60 (adequate general anesthesia for a surgery), where values below 40 represent a deep hypnotic state. The BIS index is known in the art and the main variables incorporated in the algorithm are the frequency and power spectrum of the EEG, the amount of burst suppression, and the degree of synchronization of the EEG. The US Food and Drug Administration approved BIS sensors in 1996.

Another marker that can be used is also routinely monitored during general anesthesia. The Patient State Index (PSI) uses a combination of quantitative EEG parameters described as sensitive to changes during anaesthesia but insensitive to different anaesthetic regimens and has been notably disclosed by Drover and Ortega (Best Pract Res Clin Anaesthesiol. 2006 Mar;20(1):121-8). Devices calculating this index are currently commercialized by Masimo Corporation (Irvine, California, USA).

One can also cite Spectral Entropy (Vakkuri et al., Acta Anaesthesiologica Scandinavica 2004;48(2):145-53) which provides, as does BIS, a score between 0 and 100.

Such markers are linked to the depth of anesthesia, using EEG acquired signals. The inventors propose to use the alpha power, computed from the EEG power spectral density (PSD) extracted from the EEG data.

The alpha wave frequency information can be extracted by

- Filtering EEG data
- Computing EEG power for each electrode using the fast Fourier Transform (FFT) to obtain EEG power spectral density (PSD)
- Optionally averaging the PSD for the multiple electrodes if multiple electrodes are used, to obtain a final PSD
- Obtaining alpha power ($\alpha$BP) by integrating the EEG power spectral density across the 8-13 Hz (or potentially 7.5-13 Hz) values.

It is reminded that the power spectral density is a measure of the power distribution across different frequencies in an EEG signal. Although one of skill in the art is aware of several methods to calculate PSD from EEG data, a widely used method is the Welch's method.

The EEG signal would be processed, in particular by filtering the data to remove frequencies outside the range of interest, removing or interpolating bad channels, and detrending or normalizing the data. As the studied signal is obtained from a limited time window (an epoch, as indicated above), a window function, such as the Hamming, Hanning, or Blackman window, is applied to reduce spectral leakage and improve the spectral resolution of the PSD estimate.

The Fast Fourier Transform (FFT) can then be applied to each windowed epoch to estimate the power spectrum. The power spectrum is the magnitude squared of the complex FFT coefficients, and represents the distribution of power across different frequencies.

Other methods could be envisaged for analyzing EEG data besides FFT, such as wavelet analysis, Hilbert-Huang transform, or event-related spectral perturbation (ERSP) analysis, which involves calculating a baseline spectral power for a given frequency band (for instance, the baseline could be the values just before induction of anesthesia), and comparing this baseline to the spectral power during anesthesia.

When multiple electrodes are used to acquire the EEG data, the PSD calculated for each electrode can then be averaged to obtain the final PSD which is used to compute the alpha power ($\alpha$BP).

It is also possible to use the information from another type of brain wave. One can use the power integrated (according to methods similar as the ones described above) from the beta wave (integration over the 13-30 Hz interval), the from theta wave (integration over the 4-8 Hz interval), or even from the delta wave (integration over the 0.5-4 Hz interval). In some embodiments, the power is calculated by integration over the 4-13 interval (theta and alpha waves), the 13-30 interval (alpha and beta waves) of the 4-30 interval (theta, alpha and beta waves).

It is preferable to use information associated with the alpha waves, which are a good marker of anesthesia. Consequently, use of alpha power is of very interest, or use of a power calculated with alpha and at least one other brain wave information. in other embodiments, rather than calculating a power information by combining the information from alpha and another brain wave, one can use the alpha power and the power calculated from at least one other brain wave.

The above markers of cerebral activity of the subject during anesthesia are to be calculated when the subject is under stable anesthesia.

The anesthesia is considered as stable/stationary when the rate of anesthetic drug administered to the patient is stable (and thus when the blood concentration of anesthetic drugs is stable. It is also preferred that the Mean Arterial Pressure (MAP) is above or equal to 65mm Hg.

The moment where the cerebral markers can be measured is generally reached from 10 minutes post induction of anesthesia. One can indeed consider that anesthesia induction would last for 5 minutes and that another 5 minutes would ensure that the stable anesthesia regimen is reached.

In order to use alpha power as a marker, in the methods herein disclosed, it is possible to first calculate the Spectral Edge Frequency (SEF). The SEF is calculated from the power according to frequencies. The AUC of the power spectrum is calculated first. The SEF (also called SEF95) is obtained by identifying the frequency the frequency below which 95% of the power resides. It can be determined by methods disclosed by Rampil (Anesth Analg 1983; 62: 186-192; Anesthesiology 1987; 67: 139-142). One could also envisage using the SEF90 (frequency below which 90% of the power resides).

It is possible to calculate alpha power when the Spectral Edge Frequency (SEF) is between 8-15 Hz. When it is lower, this indicates that there are no alpha waves, and one or more of BIS, PSI or Entropy indexes may be used.

Generally, alpha power is calculated on an EEC signal of less than 10 minutes, or less than 5 minutes. In order to have enough signal to obtain a representative result, it is preferred when the duration of the EEC signal is at least 30 seconds, preferably at least 1 minute. Using an EEC signal lasting between 3 and 5 minutes is adequate. It is also possible to calculate multiple alpha power values for different times of the EEC signal, and to take the mean of these values. However, since the alpha power is calculated when the subject is in a stable anesthesia state, it is foreseen that the alpha power value will essentially remain constant whenever it is calculated.

Similarly, when an index as indicated above is used, and considering the stability of anesthesia (meaning that there is little variation of these indexes), it is sufficient to take one value at a time "t" selected by the operator, or to take the mean of 2 to 10, or 2 to 5 values over a period of 5 to 10 minutes.

In some embodiment, the SF may be used as the marker of cerebral activity, notably when it is below 8 Hz (and that the alpha power thus cannot be calculated). In this embodiment, the selected value of the SEF is obtained like the indexes as indicated above.

In summary, the physiological marker of the cerebral state is based on an EEG of the subject, that has been obtained during anesthesia, and intends to reflect the depth of anesthesia of the subject, and the cerebral activity of the subject during anesthesia. The preferred markers are alpha power (or another marker that would reflect activity of the alpha wave), BIS, PSI, Entropy, or SEF. Alpha power is a preferred marker of cerebral state. Any other marker or index that would provide the same type of information can also be used.

*Cardiovascular state of the subject*

Under general anesthesia, maintaining optimal cerebral perfusion is crucial to ensure organ function, especially the brain. The conventional practice is to monitor perioperative mean arterial pressure (mAP or MAP) and verifying that it remains at an adequate value. One commonly accepted view of the blood pressure/cerebral blood flow relationship is based on the Lassen curve that assumes that there is an optimal range of cerebral perfusion, mAP typically between 60 and 150 mmHg, where the cerebral blood flow is maintained at a relatively constant level.

The physiological marker used, in the methods herein disclosed, reflects the blood pressure of the patient. One can thus the mAP or the Pulse Pressure (PP, the difference between systolic blood pressure (SBP) and diastolic blood pressure (DBP)). One can also use systolic (SBP) and diastolic (DBP) blood pressures or heart rate (HR).

Such blood pressures are measured and monitored according to methods known in the art, and widely used in anesthetic blocks.

Such monitoring may be performed using another device than the device used to monitor and/or analyze the EEG. The mAP is thus monitored and displayed on another monitor.

In some cases, mean arterial pressure is measured with a sphygmomanometer (or electronic electrocardioscope tensiometer) which uses the oscillometric measurement when the sleeve placed on the patient's forearm deflates automatically. The maximum value of the oscillation represents the mean arterial pressure. This method also allows measurement of the diastolic and systolic blood pressures, and hence of the pulse pressure.

In other cases, one can use an intraarterial catheter that directly measures the mAP. However, this technic is very invasive and is generally not used in routine.

In some cases, mAP is measured using a plethysmograph (pulse oximeter located at the tip of a finger). WO 2017/037369 and WO 2007/128518 disclose such ways of measuring mAP.

In a specific embodiment, the mAP is continuously evaluated, based on the value of the height of the dicrotic wave, continuously calculated by plethysmography, using a method comprising:

I. calculating a calibration value Calib from

(a) the value of the mean arterial pressure measured at a time t0, and
(b) the height of the dicrotic wave Vp0 measured at the time t0, wherein Calib = (value of the mean arterial pressure measured at a time t0) / Vp0 and

II. calculating the estimated value MAPest of the subject's mean arterial pressure at a time t after t0 by the formula MAPest = Calib × Vpt, wherein Vpt is the value of the dicrotic wave height obtained at the time t.

Such method for measuring mAP is disclosed in WO2019122406 and US20200390347.

One can also use the Perfusion Index (PI) which is the ratio of the pulsatile signal to the non-pulsatile signal obtained from a pulse plethysmograph. It is also conceivable to use the Pleth Variability Index (PVI), a dynamic index between 0-100 which measures the relative variability of the pleth waveform noninvasively detected from a pulse oximetry sensor.

The pressure values that are obtained during the anesthesia (preferably during the whole duration of stable anesthesia, although one can envisage using values obtained during a shorter period of time) are compared to a baseline, which is the mAP or PP that has been measured before anesthesia, when the subject is awake.

Although the measures can be performed continuously, using a plethysmograph, mAP and PP are generally, and according to current good practices, measured every now and then (generally every 5 minutes, or more often every 2-3 minutes). This allows the anesthetist to use a vasopressor to increase mAP in case of decline of such.

Consequently, the data obtained is generally a series of discrete values corresponding to the mAP or PP values obtained at different time points during anesthesia (one could also use a series of PI or PVI values).

The Root Mean Square Error (RMSE) of these values as compared to the baseline value can be calculated and can be used in the functions and methods herein disclosed. Making such calculation (calculating the RMSE with regards to a baseline, rather than with regards to the mean of the series of values) is uncommon in the art. RMSE is the square root of the average of squared differences between the measures mAP or PP values (or other values) and the baseline value. RMSE is particularly sensitive to data points that would significantly differ from other observations.

In summary, markers associated with the blood circulation and/or organ perfusion during stable anesthesia are used, notable mAP, PP, PI or PVI. mAP sBP, or PP are preferred markers, PP or sBP being the most preferred. The values used in the methods herein disclosed should reflect the deviation with regards to the baseline value measured before anesthesia. RMSE of the series of measured data is an adequate value to use in such methods and functions.

**Pharmacological markers**

Although the physiological markers are used in all methods and functions herein disclosed, it may be of interest, to detect specific risks of adverse outcomes, to also use markers associated with the pharmacological products and regimen used for the anesthesia of the subject. One will thus use data from at least one marker associated with the products administered to the subject during anesthesia.

Such markers can be obtained from the amount of drug provided to the patient during the period of stable anesthesia. Other markers associated with the amount of vasopressor and/or of any analgesic drug (such as morphine or morphine derivative to avoid pain such as alfentanil, fentanyl, remifentanil or sufentanil) can also be used in such functions.

Although propofol (injected) and sevoflurane (inhaled) are the most common anesthetic agents used for general anesthesia, and the methods herein disclosed are exemplified with these agents, other non-opioid anesthetic drugs that are administered intravenously (such as a barbiturate (in particular amobarbital, methohexital, thiamylal or thiopental), a benzodiazepine (notably diazepam, lorazepam or midazolam), etomidate or ketamine). As inhaled agents, one can also cite desflurane, isoflurane, methoxyflurane, nitrous oxide, or even enflurane, halothane or xenon.

When an injected agent is used, one marker envisaged is the mean of the TCI (Target Controlled Infusion). The TCI is linked to the brain target dose desired by the anesthetist, and after entering the desired parameters in a system, the system controls the infusion pump to inject the proper dosage, taking into account the repartition of the drug within the different body compartments and its elimination. TCI is a widely used parameter in anesthesia (Struys et al, 2016. Anesth. Analg. 122 (1): 56-69).

Different pharmacokinetics models can be used to determine the actual drug concentration in the brain. One can cite the Schnider or the Marsh model. The Marsh and Schnider models are pharmacokinetic models for determining propofol concentration in the various compartments. The Marsh model has been adapted from the Gepts model, in which the rate constants are fixed, whereas compartment volumes and clearances are weight proportional.

The Schnider model was developed during combined pharmacokinetic-pharmacodynamic modelling studies. It has fixed values for V1, V3, k13, and k31, adjusts V2, k12, and k21 for age, and adjusts k10 (elimination rate constant) according to total weight, lean body mass (LBM), and height. It was derived during a combined pharmacokinetic and pharmacodynamic study in a single set of 24 volunteers (Schnider et al, Anesthesiology, 1998, 88, 1170-82; Schnider et al, Anesthesiology, 1999, 90, 1502-16), using total body weight, age, height, and lean body mass (LBM) (calculated from total weight, gender, and height) as co-variates.

When the subject is in a stable anesthesia regimen, the infusion rate remains constant, and guarantees that the target concentration is reached and maintained.

One can also use the amount of anesthetic administered to the subject during steady-state (stable) anesthesia, divided by the duration in the block (operation room), optionally and preferably pondered by the subject's weight.

When an inhaled (gaseous) anesthetic is used, such as sevoflurane, the marker would rather be the MAC (Minimum Alveolar Concentration, described in Eger et al., 1965. Anesthesiology. 26 (6): 756-63). MAC is a regular parameter monitored in anesthesia and can be linked to the concentration of anesthetic in the air exhaled by the subject, by the formula proposed by Eiger, which also takes the age into consideration.

These two markers are markers that do not vary during the period of stable anesthesia and can be used as such in the methods and formulas herein disclosed. To facilitate combined analysis and have a function that can be use whatever anesthetic agent is used, the values of Propofol Target-Controlled Infusion (TCI) and volatile anesthetic Minimum Alveolar Concentration (MAC) can be standardized by scaling them to a range between 0 and 1.

To standardize the TCI and MAC values, one can use a Min Max function. The value to be taken into consideration would be

$$x_i' = \frac{x_i - min(X)}{max(X) - min(X)}$$

Where min(X) is the minimal value of TCI (or MAC) and max(X) is the maximal value of TCI or (MAC) as observed during anesthesia.

It is however possible to use other pharmacological markers that can be used during anesthesia, and will be adjusted by the physician in case of need.

One can cite vasopressors that are administered to the subject when hypoperfusion of the brain is detected. The vasopressor increases arterial pressure. Vasopressors are known in the art and include sympathomimetics (including adrenaline, norepinephrine (also called noradrenalin), dopamine, ephedrine...), glucocorticoids and mineralocorticoids, angiotensinamide... In hospital settings, sympathomimetics are used instead. It is not always necessary to use such agents during anesthesia, as it will depend on the evolution of the hemodynamic parameters for the subject. Furthermore, since the duration of the anesthesia may vary (depending on the type of surgical or interventional interventions), using the total amount of administered vasopressor molecule mays not be proper. It is thus advised, in the implementation of the methods and tests herein disclosed, to use, as a value for this pharmacological parameter, the cumulated dose used during the anesthesia divided by the duration of the time in the block (mean rate). Thus mean rate (which can be expressed as a "unit of weight / unit of time" or "unit of mole / unit of time") may also be pondered (divided) by the subject's weight (as a larger amount of vasopressor is needed for a heavier patient).

When an antalgic agent is used (morphine or derived thereof), it is likewise preferred to use the total amount used during intervention divided by the duration of intervention (time in the operating room), and preferably pondered by the subject's weight.

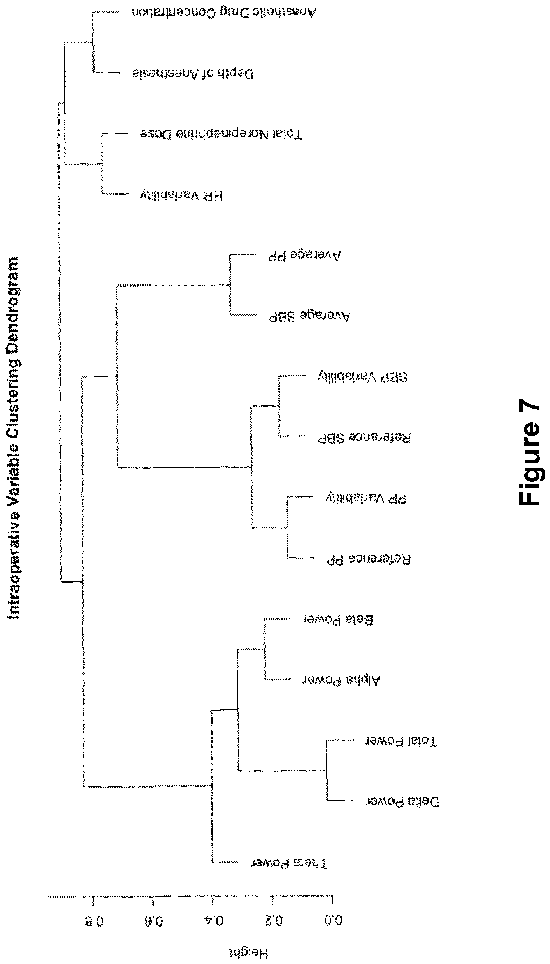

It is to be noted that Figure 7 shows clusters of markers. Markers from the same clusters can thus be considered equivalent, and it is consequently possible to substitute one marker for another in the same cluster, or for a close marker. For instance, one could use either beta or alpha power, but also total or theta power. Reference PP or RMSE of PP could be used, or Reference SBP or SBP variability (RMSE).

## Obtaining the function according to the invention

The invention also pertains to a method for obtaining a function for identifying frailty or an increased frailty risk in a patient wherein said function combines the values associated with physiological markers of said patient and optionally with pharmacological markers and/or with the age of the patient, comprising:

a) determining, in a cohort of patients having been subject to a general anesthesia, the presence of an adverse effect (cardiovascular, neurological, or other adverse effect, including death) within a given period of time (monitoring period of time), wherein physiological data and/or pharmacological data (markers) obtained during general anesthesia are known for the patients (such markers may have been derived from raw data, according to methods described above),

b) identifying by unidimensional analysis, the markers for which the values differ significantly between the groups of

i. patients having had the adverse effect during the given period of time and

ii. patients not having had the adverse effect

c) performing a logistic regression analysis to assess and ponder the independent discriminative value of the markers identified in step b) and optionally of the age of the patients for the occurrence of the adverse effect,

d) thereby obtaining the function, by combination of these identified independent factors, wherein the function allows for determining an increased risk, or an absolute risk of occurrence of the adverse effect during the given period of time for a subject.

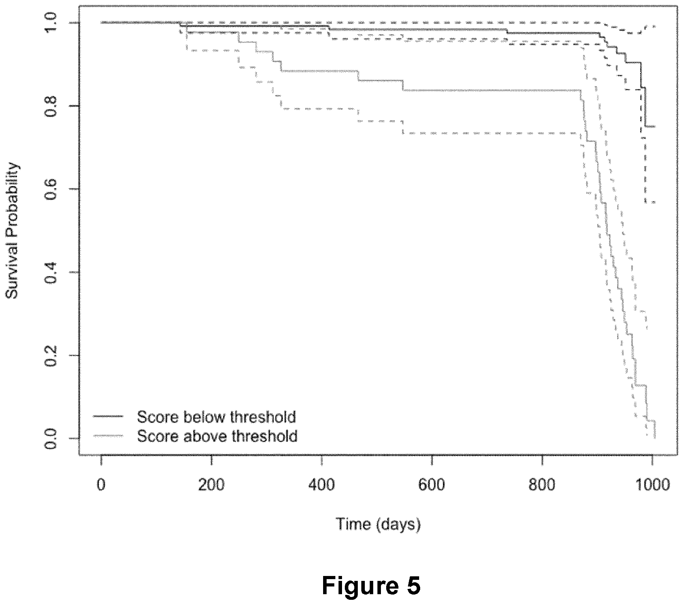

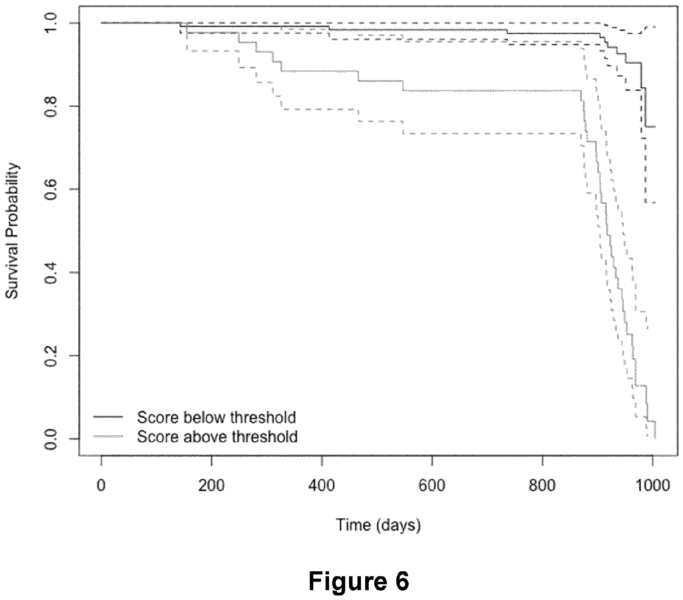

In some embodiments, the analysis in c) is a Cox analysis, providing a Cox function that provides survival (life without adverse effect) overtime. This type of function makes it possible to determine the evolution of the occurrence of the adverse effect and the divergence depending on whether the subject is positive or negative to the test (Figures 5 and 6).

In some embodiments, not all markers identified in b) are used in the analysis in c) (which leads to the regression or Cox function), and it is possible to determine the appropriate number of markers (using at least the markers for neurological and cardiovascular data), and the appropriate markers, according to what is explained in the examples (in particular determining relations between markers to avoid, as much as possible, using correlated markers, removing one marker and calculating the AUROC to determiner whether this marker adds up to the quality of the function...).

Depending on the adverse effect selected (cardiovascular adverse effect, neurological adverse effect, cardiovascular or neurological adverse effect, other adverse effect, death), the markers that will be identified in b) may vary. Some markers, that would be relevant for some adverse effects may not be relevant for others. Age may also not be relevant in some situations (for some adverse effects).

In some embodiments, the invention also pertains to a method for obtaining a function for identifying frailty or an increased frailty risk in a patient wherein said function combines the values associated with physiological markers of said patient and optionally with pharmacological markers and/or with the age of the patient, comprising:

a) determining, in a cohort of patients having been subject to a general anesthesia, the presence of an adverse effect (cardiovascular, neurological, or other adverse effect, including death) within a given period of time (monitoring period of time), wherein physiological data and/or pharmacological data (markers) obtained during general anesthesia are known for the patients (such markers may have been derived from raw data, according to methods described above),

b) identifying by unidimensional analysis, the markers for which the values differ significantly between the groups of

i. patients having had the adverse effect during the given period of time and
ii. patients not having had the adverse effect
and selecting markers,

c) scoring each marker selected in b) according to its value and assigning a discrete score to each marker
d) thereby obtaining the function as the sum of the discrete scores of c), wherein the function allows for determining an increased risk, or an absolute risk of occurrence of the adverse effect during the given period of time for a subject.

When used for determining the risk of a subject, the end result is thus obtained aggregating by summation the discrete scores of c) obtained for the markers selected in b) during anesthesia of the subject..

A discrete score (0, 1, 2...) will be assigned for each marker in c) depending on thresholds that can be identified as explained below. For each marker, "x" thresholds will define "x+1" class and the score (0, 1,...,x+1) is assigned to the marker according to the class to which it is assigned.

As an illustration, for a given marker, one can chose three thresholds "t1, t2, t3", thus defining four classes. The score "0" is assigned to the marker if its value is below t1, the score "1" is assigned if the value is between t1 and t2, the score "2" is assigned if the value is between t2 and t3 and the score "3" is assigned if the value is above t3.

In some embodiment, the same number "x" of classes are selected for each marker, meaning that the score will be comprise between "0" and "n.x" in case "n" markers are used.

In some embodiments, four classes (three thresholds) are defined for each marker. The score will thus take any integer value between "0" and "3n" when "n" markers are used.

In order to determine the threshold (cutoffs), one can use Decision Tree-based Cutoffs: Initially, decision tree algorithms can be employed to ascertain optimal cutoffs for each variable (marker). The decision tree can be constructed by iteratively splitting the data based on the variable (marker) value. The splits can be determined using the Gini impurity criterion. The complexity parameter can be based on the cross validation data to optimize the pruning process and avoid overfitting. The specific cutoffs can be determined by the splits made at each decision node and used to classify the observations into the respective outcome groups (classes).

It is also possible to use Quartile-based Scoring: For each marker, or for marker where the decision tree method does not yield distinct or clinically meaningful splits, it is possible to use the quartile distribution method. Here, variables were categorized into quartiles, and scores are assigned either in an ascending or descending manner, contingent on the clinical significance of the variable.

Ascending variables (the lower the value, the lower the score) comprise

1. Pulsed Pression Variability (score_pp_var); higher variability indicates less stable blood pressure in the patient, and it is assumed that this indicates potential cardiovascular problems.
2. Age (score_age)
3. Total Dose of norepinephrine (score_nor_total): it is assumed that need for more vasopressor support suggests that the cardiovascular system and blood vessels are more fragile.

Descending variables (the lower the value, the higher the score)

1. brain wave power, notably Alpha Band Power (score_alpha): it is assumed that lower values indicate weaker brain function, suggesting increased fragility of the brain.

2. Anesthetic Concentration (score_anesthetic_concentration): it is assumed that that a lower dose of anesthetic used to achieve a proper anesthesia depth implies that the brain is more susceptible or 'easily' sedated.

One such function is disclosed as CMEUA in the examples and is also part of the invention.

The methods herein disclosed thus make it possible to obtain

- Tests and functions rendering discrete integer outputs (end results) when the markers are assigned to a class and a score is allocated to the marker
- Tests and function rendering continuous outputs (end results) or showing occurrence of the adverse outcomes overtime when logistic regression or Cox functions are designed, using the values of the markers.

In the methods for developing such diagnostic test, one would prefer one or more of the following, in either combination:

(a) patients with post-operative complications (complications within 90 days of the anesthesia) are excluded from the cohort, to allow obtaining a function which provides inherent frailty of the patient.

(b) Patients of the cohort have been subject to non-cardiac surgery. Indeed, as hemodynamic (cardiovascular) parameters are used in the function, it is necessary to have such, which is not possible *a priori* when the patient have cardiac surgery and extra-corporal circulation.

(c) The cohort does not comprise patients with known cardiac or neurological conditions, already followed by cardiologists or neurologists for cardiac or neurological problems (including epilepsy or brain tumors); this would make it possible to detect unknown frailty.

(d) in step a), to use a test cohort comprising at least 100 patients, preferably at least 150 patients.

(e) In the cohort of a), that at least 10% of the patients, more preferably at 15% of the patients, are patients who experience an adverse effect during the monitoring period of time,

(f) The markers of step (b) are selected from

i. Physiological markers: PSI, BIS, alpha power, beta power, theta power, delta power, Entropy, SEF (markers pertaining to the cerebral state of the subject), mAP, PP, SPB, DPB, PI, PVI (markers pertaining to the hemodynamic state of the subject), Lee's score, cardiovascular history (markers pertaining to the anesthetic risk of the subject)

ii. Pharmacological markers: MAC, TCI, rate of anesthetic (which can be standardized) for assessing the amount of anesthetic provided, rate of vasopressor, rate of antalgic agent

(g) at least one physiological marker linked to the cerebral state and and least one physiological marker linked to the hemodynamic state are used in the mathematical function of c)

(h) the monitoring duration of time is at least 18 months, preferably 24 months or more, preferably 30 months or more,

(i) The method further comprises a step of validating the function obtained in c) on an independent validation cohort comprising at least 100 patients.

In order to obtain a function that is as accurate as possible, the number of patients in the cohort should be as large as possible, indicating that it preferably comprises more than 50 patients, preferably more than 100 patients, preferably more than 150 patients. As indicated, there is no upper limit for the number of patients, and the larger, the better.

### Functions according to the invention

In all embodiments, it is possible to use either the output of the function as calculated, or to normalize the output to obtain a final value comprised between 0 and 1, as this would make it easier to define predetermined values (thresholds or cutoffs) to determine whether a subject has or has not an increased frailty risk.

One can thus use the actual result "$r$" of the function combining the values as indicated above, or may be obtained after such result "$r$" has been normalized to be comprised between 0 and 1. Such output "$\underline{o}$" may be $\underline{o}=(\exp(r)/(1+\exp(\underline{r}))$. Applying such transformation to the result of the function guarantees an output between 0 and 1. In the function below, log corresponds to neperian logarithm (In).

In a first embodiment, the function is a logistic regression that makes it possible to predict occurrence of a cardiovascular adverse event (cardiovascular frailty), and thus identify patients with a higher risk of having such adverse event.

$$F1 = a1 - a2 *log(alpha1) + a3*(rmse\_pp),$$

with

- $19.5 \leq a1 \leq 22.5$, preferably $20.5 \leq a1 \leq 21.5$
- $2.5 \leq a2 \leq 3.5$, preferably $2.75 \leq a2 \leq 3.25$
- $0.04 \leq a3 \leq 0.06$

In particular, F1 = 20.906 - 3.054*log(alpha)+0.052*(rmse_pp)

This function uses, as markers / variables, the alpha power (alpha, that has been transformed to take the log value, and the RMSE of the Pulse Pressure. (rmse_pp). This functions thus uses two physiological markers, representing the cerebral activity (alpha power, obtained from an EEG), and the hemodynamic activity (Pulse Power, obtained from a cuff). It provides continuous outputs.

The AUROC (Area Under Receiving Operating Curve) for this function is AUC = 0.9247, with a PPV = 0.297 and a NPV = 0.999 (Sensitivity: 0.778, Specificity: 0.999), using a cutoff at -2.887.

It is also possible to normalize the function, as indicated above, to obtain a final value between 0 and 1. In this case, the cutoff value is 0.053.

In view of the NPV, this means that 99.9% of the patients with a result below the cutoff will not present a cardiovascular adverse event. The function makes it possible to identify patients with no risk, and an decreased risk (as compared to the patients above the cutoff) of having a cardiovascular adverse effect.

In another embodiment, the function makes it possible to predict occurrence of a neurocognitive decline adverse event (neurological frailty), and thus identify patients with a higher risk of having such adverse event.

$$F2 = b1 + b2*(age) - b3*log(alpha) + b4*(rmse\_pp) + b5*log(dose\_nor\_total),$$

with

- $16.5 \leq b1 \leq 19.5$, preferably $17.5 \leq b1 \leq 18.5$
- $0.060 \leq b2 \leq 077$, preferably $0.065 \leq b2 \leq 0.072$
- $3.10 \leq b3 \leq 3.25$, preferably $3.15 \leq b3 \leq 3.20$
- $0.060 \leq b4 \leq 0.070$, preferably $0.62 \leq b4 \leq 0.067$
- $0.20 \leq b5 \leq 0.35$, preferably $0.25 \leq b5 \leq 0.31$

In particular, F2 = 18.237 + 0.0686*(age) - 3.184*log(alpha) + 0.0646*(rmse_pp) + 0.286*log(dose_nor_total)

This function uses the age (in years), the alpha power information transformed to take the log value (log(alpha)), the RMSE of the Pulse Pressure (rmse_pp), and the total dose of noradrenaline provided to the patient (pondered by the time in the block and the subject's weight), as indicated above, transformed to take the log value (log(dose_nor total)).

The AUROC (Area Under Receiving Operating Curve) for this function is AUC = 0.953, with a PPV = 0.826 and a NPV = 0.999 (Sensitivity: 0.999, Specificity: 0.966), using a cutoff at -1,0790.

It is also possible to normalize the function, as indicated above, to obtain a final value between 0 and 1. In this case, the cutoff value is 0.250.

This function makes it possible to identify patients with no risk, and an decreased risk (as compared to the patients above the cutoff) of having a neurological adverse effect.

In another embodiment, the function is a logistic regression that makes it possible to predict occurrence of an adverse event (cardiovascular and/or cognitive decline and/or death, thus revealing frailty), and thus identify patients with a higher risk of having such adverse event.

$$F3 = c1 - c2*log(alpha) + c3*(rmse\_pp) + c4*(age) + c5*log(dose\_nor\_total)$$
$$+ c6*(anesthetic\_concentration),$$

with

- $16.5 \leq c1 \leq 19.5$, preferably $17.5 \leq c1 \leq 18.5$
- $3.10 \leq c2 \leq 3.25$, preferably $3.15 \leq c2 \leq 3.20$
- $0.060 \leq c3 \leq 0.070$, preferably $0.62 \leq c3 \leq 0.067$
- $0.060 \leq c4 \leq 0.077$, preferably $0.065 \leq c4 \leq 0.072$

- 0.30 ≤ c5 ≤ 0.40, preferably 0.32 ≤ c5 ≤ 0.38

In particular, F3 = 18.0269 - 3.1827*log(alpha) + 0.0655*(rmse_pp) + 0.0679*(age) + 0.3218*log(dose_nor_total) + 0.3543*(anesthetic_concentration)

This function uses the age (in years), the alpha power information transformed to take the log value (log(alpha)), the RMSE of the Pulse Pressure (rmse_pp), and the total dose of noradrenaline provided to the patient (pondered by the time in the operative room and the weight), as indicated above, transformed to take the log value (log(dose_nor_total)) and the anesthetic concentration (TCI or MAC, standardized as described above).

Using the same markers, a Cox function was obtained, and the Kaplan-Meier survival curve is shown on Figure 5 (the survival corresponding to the absence of occurrence of adverse event).

The AUROC (Area Under Receiving Operating Curve) for this function is AUC = 0.934, with a PPV = 0.872 and a NPV = 0.957 (Sensitivity: 0.891, Specificity: 0.949), using a cutoff at -0,5248.

It is also possible to normalize the function, as indicated above, to obtain a final value between 0 and 1. In this case, the cutoff value is 0. 37.

It is also possible to use two cutoffs (to increase NPV and PPV). Such cutoffs may be (for a normalized function) 0.7 (which provides a 0.95 PPV, Sensitivity = 0.74 and Specificity = 0.98) and 0.39 (which provides a 0.95 NPV, Sensitivity = 0.87 and Specificity = 0.95). Using these two cutoffs, patients above 0.7 can be said to have a higher (and potentially an absolute) frailty risk, while patients below 0.39 can be said to have a lower (and potentially no) frailty risk. For patients between the cutoffs, one can look at other extrinsic evidence (age, chronic diseases) to perform further cardiologic or neurologic investigations. Using the cohort of the examples, it was shown that only 6.13% of the patients were in the gray area. It thus makes sense to use these kinds of cutoffs. 89% of the patients were correctly classified, when the two cutoffs were used. It is reminded that one of skill in the art may change the cutoffs values, if desiring to emphasize reduction of false negative or of false positive.

In another embodiment, the function uses, for each marker, scores from 0-3 assigned as explained above, using cutoffs for each marker value. This function makes it possible to predict occurrence of an adverse event (cardiovascular and/or cognitive decline and/or death, thus revealing frailty), and thus identify patients with a higher risk of having such adverse event.

$$F4 = -d1 + d2×(score\_alpha) + d3×(score\_pp\_var) + d4×(score\_age) + d5×(score\_nor\_total) + d6×(score\_anesthetic\_concentration),$$

with

- 6 ≤ d1 ≤ 8, preferably 6.5 ≤ d1 ≤ 7.5
- 2.4 ≤ d2 ≤ 3.0, preferably 2.5 ≤ d2 ≤ 2.9
- 0.50 ≤ d3 ≤ 0.70, preferably 0.55 ≤ d3 ≤ 0.65
- 0.9 ≤ d4 ≤ 1.1, preferably 0.95 ≤ d4 ≤ 1.05
- 0.35 ≤ d5 ≤ 0.50, preferably 0.40 ≤ d5 ≤ 0.45
- 0.025 ≤ d6 ≤ 0.035, preferably 0.029 ≤ d6 ≤ 0.033

In particular, F4 = -6.933 + 2.702×(score_alpha) + 0.617×(score_pp_var) + 0.997×(score_age) + 0.417×(score_nor_total) + 0.0313×(score_anesthetic_concentration)

This function uses the age (in years), the alpha power information, the Pulse Pressure information (RMSE), the total dose of noradrenaline provided to the patient (pondered by the time in the operative room and the weight), and the anesthetic concentration (TCI or MAC, standardized as described above).

Using the same markers, a Cox function was obtained, and the Kaplan-Meier survival curve is shown on Figure 6 (the survival corresponding to the absence of occurrence of adverse event).

To assign the scores, as described above, one can use the scores described in Table 1.

Table 1. cutoffs for assigning scores to markers

| Marker | Cutoff 1 | Cutoff 2 | Cutoff 3 |
|---|---|---|---|
| Pulse Pressure | 36.86085 | 39.67269 | 43.60605 |
| Noradrenaline (log) | 2.2 | 4 | 4. 5 |
| Alpha power (log) | 6.5 | 7.2 | 8.7 |
| age | 50 | 64 | 74 |

(continued)

| Marker | Cutoff 1 | Cutoff 2 | Cutoff 3 |
|---|---|---|---|
| Anesthetic (scaled) TCI MAC | 0.4886 0.5182 | 0.5076 0.5917 | 0.6136 0.6971 |

The AUROC (Area Under Receiving Operating Curve) for this function is AUC = 0.914, with a PPV = 0.860 and a NPV = 0.917 (Sensitivity: 0.787, Specificity: 0.949), using a cutoff at -0,340.

It is also possible to normalize the function, as indicated above, to obtain a final value between 0 and 1. In this case, the cutoff value is 0.4158.

This function has a high NPV, meaning that it allows to exclude from future consideration the patients for which the end value is below the cutoff. Further investigation (by a cardiologist or a neurologist) can be performed for the patients with an end value above the cutoff.

It is also possible to use two cutoffs (to increase NPV and PPV). Such cutoffs may be (for a normalized function) 0.59 (which provides a 0.95 PPV, Sensitivity = 0.74 and Specificity = 0.98) and 0.1 (which provides a 0.95 NPV, Sensitivity = 0.91 and Specificity = 0.66). Using these two cutoffs, patients above 0.59 can be said to have a higher (and potentially an absolute) frailty risk, while patients below 0.1 can be said to have a lower (and potentially no) frailty risk. For patients between the cutoffs, one can look at other extrinsic evidence (age, chronic diseases) to perform further cardiologic or neurologic investigations. Using the cohort of the examples, it was shown that 27.44% of the patients were in the gray area. 69% of the patients were correctly classified, when the two cutoffs were used. It is reminded that one of skill in the art may change the cutoffs values, if desiring to emphasize reduction of false negative or of false positive. With a median age of patients in gray area being: 71 years, most of them would be proposed a check-up (a cardiac check-up is indeed suggested for patients over 60).

In another embodiment, the function is a logistic regression that makes it possible to predict occurrence of an adverse event (cardiovascular and/or cognitive decline and/or death, thus revealing frailty), and thus identify patients with a higher risk of having such adverse event.

$$F5 = e1 - e2*\log(alpha) + e3*(rmse\_pp) + e4*\log(dose\_nor\_total) +$$
$$e5*(cv\_history\_pre) + e6*(age),$$

with

- $16.5 \leq e1 \leq 19.5$, preferably $18.0 \leq e1 \leq 18.5$
- $3.05 \leq e2 \leq 3.25$, preferably $3.10 \leq e2 \leq 3.20$
- $0.050 \leq e3 \leq 0.060$, preferably $0.52 \leq e3 \leq 0.057$
- $0.25 \leq e4 \leq 0.35$, preferably $0.27 \leq e4 \leq 0.32$
- $1.10 \leq e5 \leq 1.20$, preferably $1.12 \leq e5 \leq 1.16$
- $0.06 \leq e6 \leq 0.07$, preferably $0.06 \leq e6 \leq 0.065$

In particular, F5 = 18.256 - 3.159xlog(alpha) + 0.0547x(rmse_pp) + 0.295xlog(dose_nor_total) + 1.144x(cv_history_pre) + 0.0637x(age)

This function uses the age (in years), the alpha power information transformed to take the log value (log(alpha)), the RMSE of the Pulse Pressure (rmse_pp), the total dose of noradrenaline provided to the patient (pondered by the time in the operative room and the weight), as indicated above, transformed to take the log value (log(dose_nor_total)) and the past cardiovascular history (cv_history_pre; binary value: 0 if no cardiovascular history, 1 if presence of past cardiovascular event).

The AUROC (Area Under Receiving Operating Curve) for this function is AUC = 0.948, with a PPV = 0.84 and a NPV = 0.96 (Sensitivity: 0.93, Specificity: 0.91), using a cutoff at -0,8263.

It is also possible to normalize the function, as indicated above, to obtain a final value between 0 and 1. In this case, the cutoff value is 0.30.

It is also possible to use two cutoffs (to increase NPV and PPV). Such cutoffs may be (for a normalized function) 0.82 (which provides a 0.95 PPV, Sensitivity = 0.70 and Specificity = 0.98) and 0.39 (which provides a 0.95 NPV, Sensitivity = 0.87 and Specificity = 0.94). Using these two cutoffs, patients above 0.82 can be said to have a higher (and potentially an absolute) frailty risk, while patients below 0.39 can be said to have a lower (and potentially no) frailty risk. For patients between the cutoffs, one can look at other extrinsic evidence (age, chronic diseases) to perform further cardiologic or neurologic investigations. Using the cohort of the examples, it was shown that only 7.98% of the patients were in the gray area. It thus makes sense to use these kinds of cutoffs. More than 87% of the patients were correctly classified, when the two cutoffs were used. It is reminded that one of skill in the art may change the cutoffs values, if desiring to emphasize reduction of false negative or of false positive.

It is thus possible to use various markers, providing information as to the cerebral and the hemodynamical states of the subject during anesthesia, and optionally other markers associated with the products used for anesthesia (amount of anesthetic and/or of vasopressor used during the stable anesthesia) and the age of the subject, to obtain formulas that can predict increased risk frailty (either systemic or specific to a certain type of adverse effect), or lower risk of frailty.

One of skill in the art can design other formulas, using other markers that the ones exemplified above, to obtain the same information. It is worth noting that the AUROC of the above function is quite high, and reflects the "good quality" of the tests. These illustrations show that, as demonstrated by the inventors, anesthesia can be used as a revelator of a frailty in a subject, and that the reaction of the subject to anesthesia (as shown by the variation of the cerebral and hemodynamic parameters during anesthesia) is a marker of his frailty. As shown in numerous illustrations above, combining the information obtained during anesthesia can be used to establish a diagnosis (of frailty) / prognosis (of occurrence of an adverse effect) for a subject.

It is to be noted that one of skill in the art can determine the nature of the cutoffs that are deemed informative enough. Indeed, if a higher PPV is sought, the function will be more stringent, to avoid obtaining false positive. Alternatively, it is possible to choose cutoffs which increase the NPV, to make sure that there are very little false negative patient in the group of patients considered "not at risk". It is also possible to use two cutoffs (one giving a good PPV, to identify patients at risk (above this cutoff), and another one with a good NPV to identify patients "not at risk (below this cutoff)). When the end result of a patient is between these cutoffs, the patient is in a "grey zone". In this case, the age of the patient may be taken into consideration, and the physician may address this patient to a cardiologist for a cardiac check-up if the patient is 60 or above, and to a neurologist for a neurologic check-up if the patient is 70 or above or 75 or above.

The method can be used first to determine general frailty, using one function, then the status of the subject can be refined by using a function providing cardiovascular or neurological frailty. If both functions do not give any risk, other frailty type (liver, renal) may be looked at.

## Further steps / Treatments

Once frailty has been diagnosed in a patient, the physician can start further investigation.

In particular, the physician can determine whether the patient has a latent cardiovascular condition, neurological condition, liver condition, renal condition, or cancer.

Preferably, presence of a cardiovascular condition is determined by electrocardiograms, scanner, MRI, echography, or detection of biomarkers associated with cardiovascular diseases (see also below).

Presence of neurological condition may be determined by performing specific cognitive tests aimed at detecting presence of neurological diseases and/or performing clinical testing such as scanners, electroencephalograms, MRI (see also below).

Presence of a liver condition may be determined notably by an *ex vivo* diagnosis method for the presence of liver fibrosis, steatosis, cirrhosis (such as the methods disclosed in WO 02/16949, WO 2006/082522, WO 2006/103570, WO 2013/079711, WO 2018/050804, WO 2019/076830 or WO 2020/021058), or by liver ultrasound, liver stiffness measurement or MRI.

Presence of a renal condition may be determined by assessing the level of creatinine or the renal clearance,

Presence of a cancer may be determined by scanner, MRI or measurement of cancer biomarkers in the blood, urine, cerebrospinal fluid or genome of the patient

When a function that provides an increased cardiovascular frailty risk is used, the physician shall perform specific tests aimed at detecting presence of cardiovascular diseases. Such tests include physical examination to reveal signs such as high blood pressure, abnormal heart sounds, or peripheral artery disease, performing an electrocardiogram (ECG to detect irregular rhythms, heart attacks, and other heart abnormalities), or an echocardiography (to obtain detailed images of the heart's structure and function, helping to diagnose conditions like valvular heart disease, heart failure, and congenital heart defects), performing cardiac stress testing (stress tests or pharmacological stress tests to evaluate how the heart responds to physical exertion to help diagnose coronary artery disease), have the patient have chest X-ray, which may reveal abnormalities in the heart, lungs, or blood vessels that can be indicative of heart disease, conduct cardiac catheterization (angiography), to identify blockages or narrowing in the blood vessels, or coronary computed tomography angiography (CCTA) to produce detailed images of the coronary arteries, aiding in the diagnosis of coronary artery disease, perform Magnetic Resonance Imaging (MRI), blood tests (measuring various markers, including cholesterol levels, triglycerides, and cardiac biomarkers (troponin and B-type natriuretic peptide), which can indicate heart disease and assess the risk of a heart attack), helping to diagnose arrhythmias and other irregular heart rhythms, perform electrophysiology studies (EPS) to assess the heart's electrical conduction system and helps diagnose and treat arrhythmias, carotid ultrasound to assess the presence of atherosclerosis and the risk of stroke, conduct Peripheral Arterial Disease (PAD) testing by tests such like ankle-brachial index (ABI) and Doppler ultrasound, conduct genetic testing to identify inherited cardiovascular conditions, such as familial hypercholesterolemia or long QT syndrome, Computed Tomography (CT) Scans or provide and have the patient wear a Holter monitor or an Event monitor to record the heart's electrical activity over an extended period.

Treatment will thus be adapted to the exact condition of the patient (use of statins, of hypotensive medications, stent implantation, angioplasty, cardiac by-pass, or even cardiac graft). Treatments include

Cholesterol-Lowering Medications: Statins and other lipid-lowering drugs to manage high cholesterol levels.
Blood Pressure Medications: Antihypertensive drugs to control high blood pressure.
Antiplatelet Medications: Aspirin and other antiplatelet agents to reduce the risk of blood clots.
Anticoagulants: Blood thinners to prevent or treat blood clots in conditions like atrial fibrillation or deep vein thrombosis.
Medications to Manage Heart Rhythm: Antiarrhythmic drugs to control irregular heart rhythms.
Angioplasty and Stent Placement
Coronary Artery Bypass Surgery (CABG)
Catheter Ablation
Device Implantation: Pacemaker, Implantable Cardioverter-Defibrillator (ICD), Cardiac Resynchronization Therapy (CRT) Devices
Cardiac Rehabilitation: Supervised exercise programs and education.
Heart Transplant and Ventricular Assist Devices (VADs)
Stroke Treatments: Clot-busting medications (thrombolytics) or mechanical thrombectomy for ischemic strokes
Risk Factor Management: Treating and controlling conditions like diabetes, obesity, and metabolic syndrome that contribute to cardiovascular disease.

Thus, the function and methods allow for specific follow-up, characterization and/or treatment of patients identified as being at risk of cardiovascular disease.

When a function that provides an increased neurologic frailty risk is used, the physician shall perform specific tests aimed at detecting presence of neurological diseases, such as Alzheimer's disease, attention deficit disorder, dementia with Lewy bodies disease, early onset dementia, epilepsy-related cognitive dysfunction, fronto-temporal dementia, mild cognitive impairment, normal pressure hydrocephalus. These tests include assessing cognitive function using standardized tests like the Mini-Mental State Examination (MMSE) or Montreal Cognitive Assessment (MoCA), performing a physical examination to assess neurological signs and symptoms, performing brain imaging (Magnetic Resonance Imaging, Positron Emission Tomography (PET) scans, Single Photon Emission Computed Tomography (SPECT) scans), making biomarker analysis (Cerebrospinal fluid (CSF) analysis for detecting specific proteins like amyloid-beta and tau, which can be indicative of Alzheimer's disease or other neurodegenerative disorders, looking for blood-based biomarkers such as neurofilament light chain (NfL) and beta-amyloid), perform genetic testing to identify gene mutations associated with hereditary forms of neurodegenerative diseases like familial Alzheimer's disease or Huntington's disease, conduct neuropsychological assessment (in-depth cognitive and psychological testing to evaluate memory, language, problem-solving, and other cognitive functions), making electroencephalography (EEG) analysis to detect abnormal brain wave patterns associated with certain neurological conditions, such as epilepsy or frontotemporal dementia, obtaining data from functional imaging (fMRI and resting-state fMRI to show how the brain functions in different regions and networks, helping to identify abnormalities in cognitive decline), detecting tau protein aggregates or the presence of amyloid plaques in the brain by Tau-PET imaging or Amyloid-PET imaging.

Treatments for cognitive decline include

-    For Alzheimer's Disease

Medications: Cholinesterase inhibitors (e.g., donepezil, rivastigmine) and N-methyl-D-aspartate (NMDA) receptor antagonists (e.g., memantine) are commonly prescribed to manage symptoms and slow cognitive decline.
Lifestyle Interventions: Encouraging a healthy diet, regular exercise, and cognitive stimulation.
Supportive Care: Providing support and assistance for daily activities.

-    For vascular dementia

Treating underlying vascular risk factors, such as high blood pressure, diabetes, and high cholesterol.
Medications to manage stroke risk and prevent further damage to blood vessels.
Cognitive rehabilitation to improve or maintain cognitive function.

- For Lewy body dementia

    Medications to manage hallucinations, sleep disturbances, and motor symptoms.
    Supportive care and lifestyle modifications.
    Physical therapy and occupational therapy to address motor symptoms.


- For frontotemporal dementia:

    Medications to manage behavioral and psychological symptoms.
    Speech and language therapy for communication difficulties.
    Supportive care and counseling for patients and caregivers.


- For Parkinson's Disease dementia

    Medications to manage motor symptoms and cognitive decline.
    Physical therapy, occupational therapy, and speech therapy.
    Deep brain stimulation (in some cases).


- For Huntington's Disease:

    Medications to manage movement disorders and psychiatric symptoms.
    Physical and occupational therapy to improve quality of life.
    Genetic counseling and support for families.


- For Mild Cognitive Impairment (MCI):

    Addressing underlying causes, such as vascular risk factors or depression.
    Cognitive training and rehabilitation.
    Monitoring for progression to dementia and adjusting treatment accordingly.


- For mixed dementia:
    Treatment may involve a combination of strategies depending on the specific combination of underlying conditions.


Thus, the function and methods allow for specific follow-up, characterization and/or treatment of patients identified as being at risk of neurological disease.

When a function that provides and increased frailty risk (cardiovascular, neurologic, death) is used, the physician shall perform the above tests to determine the actual patient's condition and adequate treatment.

The invention also relates to a method for treating a patient having frailty, comprising the steps of performing the method of diagnosis/prognosis as disclosed above and providing the patient with appropriate treatment depending on the type of frailty and the risk identified by potential further investigation.


**Implementation of the methods**

The methods herein discussed are performed *in vitro,* or *ex vivo.* They can be computer implemented, in particular when the information pertaining to anesthesia and the age of the subject are provided to one or more processor, where the one or more processor perform the transformation of the raw data and the calculation of the end value.

In a specific embodiment, the first mean is computerized. It may be an electronic spreadsheet with the formula recorded within, that provides the first index as an output when entering the various elements mentioned above. It can also be a computer program that provides the first index as an output after receipt of the various elements mentioned above.

The first means can present one or more of the following, in either combination:

- Operate within a private or public network
- Receive the inputs (values of the various elements mentioned above) from a sender that is in a remote place (i.e. they are sent to the first means from a different location that where the first means is located)
- Require the sender to identify himself before sending the inputs
- Receive the inputs (values of the various elements mentioned above) from a secure manner
- Send the output (first index) to the sender of the inputs
- Store the output in a database (possibly with a unique identifier, making it possible to assign inputs, outputs to this identifier)

- Provides the first index with further information (such as sensitivity and/or specificity and/or positive predictive value and/or negative predictive value linked to the prevalence of the condition in the population to which belongs the patient)

Is also foreseen a non-transitory computer readable storage medium, having stored thereon a computer program comprising program instructions, the computer program being loadable into a data-processing unit and adapted to cause the data-processing unit to carry out a method for calculating a first index by combining the values of a patient, calculated as herein disclosed, through a function as disclosed above, when the computer program is run by the data-processing device.

In another embodiment, the invention relates to a microprocessor comprising a computer algorithm to perform a method of determining the presence of frailty (or increased risk thereof) in a patient: providing the values of a patient, calculated as herein disclosed, and optionally the age of the patient; and combining the values through a function (as disclosed above, or obtained as disclosed above) to obtain a score (the end-result or first index) useful for presence of frailty (or increased risk thereof) in the patient.

The invention also relates to the use of the functions, devices and/or microprocessors in order to make a diagnosis of presence of frailty (or increased risk thereof) in a patient by use of the functions and comparing the result to a predetermined threshold to determine the presence of frailty (or increased risk thereof) in the patient.

The invention also includes a device for diagnosis an increased frailty risk (as defined above) in a patient, comprising a means, wherein the means provides an end result by performing a method as described above. In particular, the device comprises means for receiving the data used for performing the methods herein disclosed and means for obtaining the numerical values and/or combining such.

Said end result can be later used to determine presence of an increased frailty risk in said patient and whether it is necessary to initiate treatment or follow-up, or perform further investigations.

The above method can be computer-implemented. In this embodiment, it is possible to perform a method for determining whether a patient has an increased frailty risk (as defined above), comprising

a) requiring a sender to identify himself to a system within a public or private network, wherein the sender is generally in a place remote from the system

b) requiring the sender to fill in information related to the patient (such as social insurance number, sex, birth date, weight, height), and assigning a specific identifier to the patient in a database, (this last step may be omitted if the patient has already been recorded in the database, as can be determined, using the patient information);

c) receiving data associated with an anesthesia of the patient, wherein the data comprises information pertaining to the cerebral and to the hemodynamic states of the patient before and during anesthesia, and optionally pertaining to the pharmacological parameters of the anesthesia and of the age of the patient (it may be calculated from the birth date), wherein the values are received in a secure manner, and storing the values in the database, associated with the specific identifier of the patient

d) Optionally transforming and standardizing the data received in c) to obtain numerical values for each received information

e) combining the values obtained in d) by use of a function as disclosed herein, obtaining a result, and optionally normalizing the result

f) storing the result optionally normalized of d) in a database associated with the specific identifier and preferably the date and time of identification of the sender, thereby obtaining a database where the information related to the patient is present under the specific identifier,

g) sending the nature of the presence of an increased frailty risk to the sender, wherein the patient has an increased frailty risk if the result is above a predetermined value and the patient has no increased frailty risk if the result is below the or another predetermined value.

In a first step, a sender is required to identify himself to a server or system within a public or private network. It is preferred when the network is a private network, and when the connection to the server is encrypted. Identification of the sender can be performed using login and password, preferably through strong identification processes (such as one-time password, or using biometric or smart card identification).

Upon login to the server, the sender is required to fill-in information related to the patient (such as name, sex, birth date, height, weight, social security identification number, social insurance number) and a specific identifier is assigned to the patient in a database. If the database does not contain any entry relating to the patient, one entry is created. If an entry already exists (using the social security identification number allows ensuring the unicity of entry), such entry is activated.

The server shall then receive information pertaining to the cerebral state of the patient during anesthesia. Such information may be a raw EEG, the alpha power if already calculated from the raw EEG, the SEF, the BIS, the Entropy and/or the PSI indexes. The server shall also receive information pertaining to the hemodynamic parameters of the patient during anesthesia. This information can be recording of the mAP, of the PP, of the SBP, of the DBP and/or of the HR. Information pertaining to the pharmacological parameters of the anesthesia are also received by the server, such as the BPI or the MAC, the amount of vasopressor used, the amount of antalgic drug used and/ the amount of anesthetic drug used, the duration of anesthesia, the duration of stable anesthesia, the duration of time in the operating room.

Such information is stored in a database, associated with the specific identifier of the patient, so that such data is uniquely associated to the patient.

Exchanges between the sender's device and the server are very advantageously performed in a secure manner (encrypted exchanges).

It is to be noted that the sender may be in a remote place different from where the server is located. It is also envisaged that the information is recovered by the server, using information provided by the sender. For instance, the server may open a connection with the server of the hospital or health establishment where the anesthesia took place, using identifiers provided by the sender, to upload the required information. Alternatively, the information may be filled in by the sender.

The next step is to transform and optionally standardize the received data in numerical values for each received information. This step is performed by a processor in the server or able to use the information from the databases. As indicated above, calculating the alpha power, the SEF can be performed using the raw EEG data. The numerical value associated with BIS or MAC can be standardized according to the methods herein disclosed. The value that is associated with the hemodynamic parameters is determined according to the methods herein disclosed. The age of the patient may be obtained from the birth date of the patient. Using the duration of in the operating room, the weight of the patient, the amount of vasopressor or antalgic drug can also be obtained in a format as specified above. Such calculation may be performed by a processor in the server. Such processor may also be programed to determine the period of stable anesthesia (using the EEG, the hemodynamic parameters, it is indeed possible to identify the induction and the wakening phases), and its duration. When the numerical values to use in the function are discrete ones (i.e. when a score is to be assigned to each marker according to predetermined cutoffs), the processor shall assign the adequate scores. The numerical values are to be stored in a database, that is associated with the raw information received, and the unique identifier of the patient (as well as the date of calculus).

The next step is to combine the numerical values in a function as disclosed herein to obtain an end result, that is optionally normalized (to be between 0 and 1) as disclosed. This end result may be compared to one or more set cutoff(s) (reference value).

The end result and information pertaining to the comparison is then stored in a database associated with the identifier specific of the patient and is preferably date and time-stamped. Thereby, a database is obtained, containing identification related to the patient (patient's specific identifier), associated with the data pertaining to the anesthesia and comparison information, and optionally but preferably the intermediate numerical values and the end result.

The nature of the risk of the patient to have an adverse effect (cardiovascular and/or neurologic decline and/or death) (increased or not) is thus determined according to the methods herein disclosed.

Such information (nature of the risk of the patient) is then transmitted to the sender, optionally with the result of the function and with any other potential information of interest.

The invention also relates to a computer-implemented method for determining whether a subject has an increased frailty risk, comprising

a) Receiving inputs from a sender, wherein the inputs are data associated with an anesthesia of the patient, wherein the data comprises information pertaining to the cerebral and to the hemodynamic states of the patient before and during anesthesia, and optionally pertaining to the pharmacological parameters of the anesthesia and of the age of the patient (it may be calculated from the birth date), as well as the information pertaining to the birth date, age, height, weight and sex of the subject, wherein the inputs are received in a secure manner

b) Optionally transforming and standardizing the data received in a) to obtain numerical values for each received information, and storing the numerical value in the database

c) Performing the method as disclosed herein, to obtain an output (end-result) for the combination of the values received in a) or obtained in b)

d) optionally comparing the output to a reference value and determining the presence of an increased frailty risk

e) Sending the output to the sender and the presence of an increased risk, if determined, and optionally further information (such as sensitivity and/or specificity and/or positive predictive value and/or negative predictive value linked to the prevalence of the condition in the population to which belongs the patient)

f) Optionally storing the inputs and the output in a database (preferably with a unique identifier associated with the patient and/or with the sender, making it possible to assign inputs, outputs to this identifier)

The invention also relates to a method for obtaining the information pertaining to the presence of an increased risk of frailty in a subject, comprising

a) Sending inputs to a remote server that is in a remote place (i.e. they are sent to the first server that is in a different location than the location of the sender), preferably in a secure (encrypted) manner, wherein the inputs are data associated with an anesthesia of the patient, wherein the data comprises information pertaining to the cerebral and to the hemodynamic states of the patient before and during anesthesia, and optionally pertaining to the pharmacological parameters of the anesthesia and of the age of the patient (it may be calculated from the birth date), as well as the information pertaining to the birth date, age, height, weight and sex of the subject,

b) Having the remote server

optionally transform the data into numerical values, and
combine the values received in (a) or transformed in a function, according to the methods herein disclosed, and
obtain an output (or end result), and optionally normalize the output

c) optionally having the remote server compare the output to a reference value and determining the presence or absence of an increased frailty risk for the subject

d) Optionally having the remote server store the inputs and the output in a database (possibly with a unique identifier associated with the subject and/or with the sender, making it possible to assign inputs, outputs to this identifier)

e) receiving the output from the server and/or the information pertaining to the presence or absence of the increased frailty risk (when determined) and optionally further information (such as sensitivity and/or specificity and/or positive predictive value and/or negative predictive value linked to the prevalence of the condition in the population to which belongs the subject).

In another embodiment, the invention relates to a microprocessor comprising a computer algorithm to perform a method for calculating the presence or absence of an increased frailty risk from information associated with an anesthesia of a subject, as herein disclosed, through a method as disclosed above.

The invention also relates to the use of the functions, devices and/or microprocessors in order to assess presence or absence of an increased frailty risk in a subject by use of the methods herein disclosed.

## FIGURES

Figure 1: Study follow up from inclusion (D-1) to time of point (Y+2.5).
Figure 2: ROC Curves for the Clinical and Augmented Models
Figure 3: ROC curves representing the predictive performance of the CMEUA score across Favorable Prognosis, Cognitive Decline, Cardiovascular event, and Mortality (curves from left to right, when looking at sensitivity 0.2)
Figure 4: Flowchart of the follow up of patients
Figure 5: Kaplan-Meier survival curve for adverse outcome (cardiovascular, neurologic or death), using the same markers as logistic regression F3.
Figure 6: Kaplan-Meier survival curve for adverse outcome (cardiovascular, neurologic or death), using the same markers as logistic regression F4 (using score of markers).

## EXAMPLES

Example 1. Material and methods

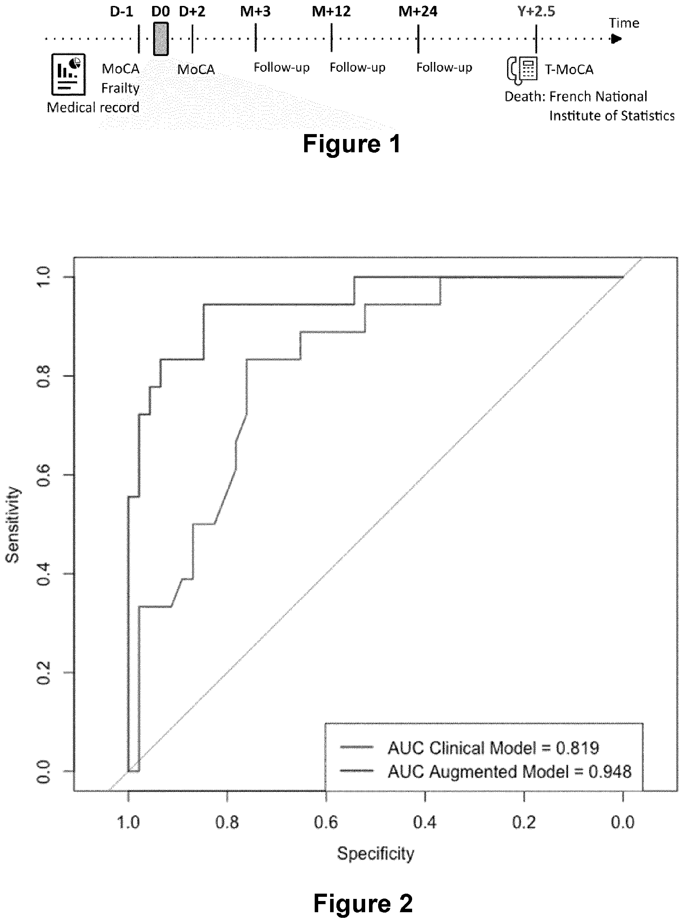

*Study Design and Patient Selection Criteria*: a single-center longitudinal cohort study was conducted at Paris' Lariboisiere Hospital, France, from February 2020 to June 2023. Patients 18 or older undergoing major orthopedic or interventional neuroradiology elective surgeries under GA were eligible. Follow-up evaluations were systematically carried out at 3, 12, and 24 months after the surgery, as illustrated in Figure 1. To ensure the reliability of the findings regarding clinical outcomes, which include mortality, cardiovascular events, and cognitive decline, any patient who encountered these complications within the first 90 days following the surgery was excluded. This measure was taken to mitigate the confounding impact of the surgical procedure itself on the outcomes measured. For the >2-year follow-up, participants were required to have not undergone any surgical procedures within the preceding year and to have preserved language abilities, as these were essential for the cognitive assessments conducted in our study.

*Anesthetia management*: General anesthesia was standardized across all procedures. Induction was achieved with sufentanil or remifentanil, followed by atracurium besilate for muscle relaxation. Blood pressure was managed to maintain at 80% of baseline using norepinephrine. Maintenance agents included sevoflurane, desflurane, or propofol, with the choice at the anesthesiologist's discretion. Monitoring protocols aimed to maintain patient state index and spectral edge frequency within specified ranges to prevent burst suppression. Detailed descriptions of the anesthetic drugs, dosages, and monitoring parameters are described below. GA protocols were standardized for both induction and maintenance phases. For induction, sufentanil at a dosage of 0.2 $\mu$g/kg was used in orthopedic surgery and remifentanil targeted at 3-5 ng/ml, titrated according to Minto's model, for interventional neuroradiology procedures. This was accompanied by the non-depolarizing neuromuscular blocking agent, atracurium besilate. Hemodynamic stability was managed by maintaining blood pressure at 80% of baseline levels, facilitated by the administration of diluted norepinephrine at a concentration of 5 $\mu$g/mL. Maintenance of GA was at the discretion of the attending anaesthesiologist, with the options including sevoflurane, desflurane, or propofol. When propofol was selected, target-controlled infusion (TCI) was employed using automated syringe pumps following the Schnider model. For halogenated agents, the minimum alveolar concentration (MAC) was maintained at 1%. The use of ketamine during induction was optional, based on the attending anaesthesiologist's judgment. According to departmental guidelines, intraoperative monitoring aimed to keep the patient state index (PSI) within the range of 25 to 50 and the spectral edge frequency (SEF95) between 8 and 15 Hz, with a specific emphasis on avoiding burst suppression.

*Data collection methodology*: Perioperative data were systematically gathered from digital patient records and integrated monitoring systems, which included medical history, hemodynamic parameters, cognitive evaluations, and prospective tracking of poor prognosis. To ensure the objectivity of the analysis, all data assessments were conducted with the researchers blinded to patient outcomes. This blinding protocol was essential to maintain impartiality and prevent bias in the evaluation of the data.

1. Preoperative Data Collection: Medical characteristics, socio-demographics were gathered using the digital patient record.
2. Intraoperative Data Collection: Brain function variables were assessed through the analysis of the frontal EEG using the SedLine Brain Function Monitor. Hemodynamic intraoperative data included Heart Rate (HR), Systolic Blood Pressure (SBP), Diastolic Blood Pressure (DBP), Mean Arterial Pressure (MAP), and Pulsed Pressure (PP). Other data points included Pulse Oxygen Saturation (SpO2), temperature, and Expired CO2 Fraction (EtCO2). Drug administration, including the concentration of halogenic drugs, propofol infusion rate, and norepinephrine infusion rate, was assessed as well. All these data were extracted as numeric variables from the Intellispace Critical Care and Anaesthesia digital record (ICCA, Philips Healthcare).
3. Postoperative Data Collection: Monitoring for postoperative complications, medical follow-up, and poor prognosis was done prospectively using the electronic medical record for the 3, 12, and 24-month follow-up periods.

Patient health assessments: Preoperative evaluations covered a spectrum of health metrics: frailty via the Clinical Frailty Scale, independence through the Instrumental Activity of Daily Living (IADL) Scale, muscle strength assessment with a dynamometer, pain quantification using the Numerical Rating Scale (NRS), and prognostic indicators.

This involved assessments for frailty with the Clinical Frailty Score (CFS), dependency with the Instrumental Activities of Daily Living (IADL) score, sarcopenia with handgrip strength measured with a dynamometer, subjective cognitive complaint at baseline and 2.5 years (in response to the question, "Have you noticed a problem with thinking or memory in daily life?"), pain and anxiety with the numeric rating scale (NRS) at baseline and 2.5 years. Postoperative complications and poor prognosis were collected prospectively. All new medical and drug history was recorded.

*Analysis of EEG, hemodynamic, drugs and other variables*

EEG monitoring: EEG recordings provided intraoperative insights into cerebral function under anesthesia. Stable periods for EEG analysis were selected as those occurring 15 minutes after induction, with a consistent 5-minute timeframe where the concentration of hypnotic drugs was maintained and MAP exceeded 70 mmHg. A spectrogram, which graphically represents the variation in power across different EEG frequency bands over time, aided in identifying suitable EEG segments for power spectral analysis. The depth of anesthesia was assessed using the Patient State Index (PSI), averaged over these identified stable EEG periods.

The spectrogram was computed after band-pass filtering through the [0.5, 25] Hz range using the Chronux toolbox in Matlab [Bokil et al. J Neurosci Methods. 2010;192:146-151].

EEG data segments devoid of burst suppression or transitioning features (such as burst or alpha suppression or emerging) were visually selected on the spectrogram during a period of at least 10 minutes of stable anesthesia. A contiguous 5-minute window of EEG data was selected for analysis, ensuring the window was free of artifacts, that the mean arterial pressure was above 70 mmHg, and that the hypnosis concentration was stable for 10 minutes before and after the selected period of analysis. The Power Spectral Density was computed using Multitaper methods implemented in the Chronux library [Bokil et al. J Neurosci Methods. 2010;192:146-151]. Power over the frequency bands of interest (delta = [0.5, 4] Hz; theta = [4, 8] Hz; alpha = [8, 13] Hz; low alpha = [8, 10] Hz; high alpha = [10, 13] Hz; and beta = [13, 25] Hz) were then computed.

Drug analysis: Hypnotic drug concentrations were examined during stable anesthesia, with Propofol Target-Controlled Infusion (TCI) levels and volatile anesthetic Minimum Alveolar Concentration (MAC) normalized between 0 and 1 via min-max scaling for comparative analysis. Norepinephrine infusion rates were evaluated by adjusting the total dosage according to duration in the operative room.

Hemodynamic variable analysis: continuous monitoring of key indices, including MAP, systolic (SBP) and diastolic (DBP) blood pressures, pulse pressure (PP), and heart rate (HR), was undertaken to evaluate vascular stability. Intraoperative hypotension (IoH) was defined as any duration during the operation where MAP remained below 65 mmHg or experienced a 20% reduction from the 'reference' value. The 'reference' value is established by the initial hemodynamic measurements captured in the operating room before the administration of anesthesia. Conversely, the 'average' value denotes the mean of these measurements throughout the course of the surgery. The extent of variability for these parameters was quantified using the Root Mean Square Error (RMSE), which offers a measure of the average fluctuation of each variable from its 'reference' throughout the procedure. Several hemodynamic variables were assessed, namely mean arterial pressure (MAP), systolic (SBP) and diastolic (DBP) blood pressures, pulsed pressure (PP), and heart rate (HR). Intraoperative hypotension (IoH) was characterized by a MAP falling below 65 mmHg or a decrease of 20% from its baseline value. Baseline value was defined as the first value of hemodynamics parameters before anesthesia start, the average value as the mean of all values during the procedure.

To quantify the deviations of these hemodynamic variables from their baseline, the Root Mean Square Error (RMSE) was employed. For each of the variables MAP, SBP, DBP, PP, and HR, variability using RMSE was obtained using the formula:

$$RMSE = \sqrt{\frac{1}{n} \sum_{i=1}^{n} (x_i - x_{baseline})^2}$$

where $x_i$ represents the value of the variable at the $i^{th}$ time point, $x_{baseline}$ is the baseline value of the variable (i.e., the value collected at the beginning of the procedure before anesthesia), and n is the total number of measurements taken. The RMSE thus provided a measure of the average magnitude by which each hemodynamic variable deviated from its baseline value throughout the procedure.

*Outcome measures and criteria*

Composite Outcome of Poor Prognosis: The study's primary endpoint for 'poor prognosis' was a composite of mortality, major cardiovascular events, or significant cognitive decline. For mortality, verification was obtained from the French National Institute of Statistics (INSEE) death registry. The cardiovascular component included documented cases of stroke or myocardial infarction. Cognitive decline was rigorously assessed utilizing the Montreal Cognitive Assessment (MoCA) and its telephonic counterpart (T-MoCA), which were adjusted to a unified scale of 22 points. Baseline cognitive performance was compared with follow-up evaluations conducted at hospital discharge and again at 2.5 years. A decline in cognitive scores equivalent to or exceeding one standard deviation from the individual's baseline was classified as cognitive deterioration.

Criteria for Favorable Prognosis: Conversely, a 'favorable prognosis' was characterized by the absence of mortality, cardiovascular incidents, or cognitive impairment within the 2.5-year follow-up period.

For clarity and to maintain methodological transparency, further details on the outcome measures, scoring adjustments, and normalization process are provided below.

Cognitive Decline Outcome Assessment: Cognitive assessments were performed at entry to the study, typically the day before surgery, at discharge from the hospital or on Day 3 (whichever was earlier), and 2.5 years post-surgery. The use of both MoCA and T-MoCA tools across these three timepoints allowed for a comprehensive assessment of patients' cognitive changes over time and post-surgery. The MoCA is a cognitive screening tool that takes approximately 15 minutes to administer. It evaluates multiple cognitive domains: attention and concentration, executive functions, memory, language, visuo-constructional skills, conceptual thinking, calculations, and orientation. The T-MoCA is a telephone version of the MoCA and omits the visuospatial/executive and naming component of the original MoCA, resulting in a maximum score of 22. Consistency across assessments was ensured by converting MoCA scores to a 22-point scale, by excluding the visuospatial/executive and naming component values. This was done to facilitate direct comparison with the T-MoCA scores. The use of T-MoCA was prioritized for long-term follow-up to minimize loss-to-follow-up rates. To identify cognitive decline, we calculated z-scores as follows:

$$\text{Z-score} = (\text{patient baseline score - mean baseline score from controls}) / (\text{standard}$$
$$\text{deviation (SD) from control group) [23,24]}$$

To account for the learning effect at 2.5 years, one of the three versions of the T-MoCA that hadn't been previously used for each patient was carefully selected. Large negative z-scores showed deterioration in cognitive function from baseline. Ultimately, cognitive decline at 2.5 years was defined by the presence of a subjective cognitive complaint together with a z-score at 2.5 years $\leq$ -1 SD.

*Variables Selection for Models Computation*

Step 1: the initial variable selection was informed by univariate analysis and the identification of established prognostic indicators. To delineate the relationships among variables, a correlation matrix was constructed using Pearson's correlation coefficient, which informed the development of a dendrogram through hierarchical clustering. This visual representation of variable clusters, based on their correlation coefficients, facilitated the identification of non-redundant variables, which were then chosen for the predictive models to minimize multicollinearity and maintain the multidimensional integrity of clinical assessments.

Step 2: the variable selection was refined utilizing a systematic, data-driven stepwise approach guided by the Akaike Information Criterion (AIC), implemented via the 'step' function in R. This process iteratively refined a comprehensive model by adding or subtracting variables, aiming for a model with the lowest possible AIC value-a balance indicative of optimal model parsimony and predictive power.

Employing this two-step methodological framework, two distinct models were constructed: a Clinical Model, which incorporated clinical variables exclusively, and an Augmented Model, which combined clinical variables with intraoperative data for enhanced prognostic capability.

*Statistical Analysis*

Significance threshold was set at 0.05. Categorical variables were reported as counts and percentages, continuous variables were described using means and standard deviations or medians with interquartile ranges, as appropriate. Comparative analyses were conducted using $\chi^2$, Fisher's exact test, Student's t-test, or Mann-Whitney U test, as appropriate. To detect potential collinearity among predictors, we calculated the Variance Inflation Factor (VIF). P-values were adjusted for multiple comparisons using the false discovery rate method [Storey J R Stat Soc Ser B Stat Methodol. 2002;64:479-498]. Missing data were addressed through multiple imputation using the MICE method. Multivariate regression analysis was applied to adjust for confounding variables.

A certain degree of missing data was expected, especially with respect to long-term cognitive and health outcome measurements. Given the occurrence of missing data in some variables (see table below), a multiple imputation strategy was employed using the Predictive Mean Matching (PMM) method. The mice package in R was utilized to generate five imputed datasets, ensuring robust and reliable statistical analyses. The imputation process was iterated 50 times for accuracy, and a seed was set for reproducibility.

For power analysis, it was aimed for 80% power with an alpha level of 0.05. An odds ratio of 2 was anticipated and a baseline probability of 40% was set for the logistic regression. It was accounted for a 0.5 cross-correlation correction for potential confounder analysis and projected a 10% loss to follow-up, leading to 150 patients.

*Primary Objective*

The primary objective of the study below was to assess the discriminative power of the Augmented Model in predicting « poor prognosis » (composite of death, cognitive decline, cardiovascular event). To this end, the Receiver Operator Curve (ROC) was employed. We juxtaposed the ROC-AUC results of the Clinical Model against those of the Augmented Model to draw comparative insights. The significance of differences between their AUCs was evaluated using the DeLong test. Secondary objective was to study the ROC-AUC of the Augmented Model for each level of the composite score using multinomial analysis. To evaluate the predictive accuracy of our metrics, the dataset was divided into a training set (70%) and a test set (30%). To gauge the inherent uncertainty and robustness of our model, 2000 bootstrap iterations were undertaken.

All the analyses were conducted using R software and the reporting is consistent with the STROBE guidelines [von Elm et al. PLoS Med. 2007;4:e296].

CMEUA Score Computation: the Continuous Metrics Evaluation under Anesthesia (CMEUA) score was developed using ten pivotal parameters identified through univariate analysis and known prognostic indicators. These parameters include age, baseline PP values, relative MAP and PP variations during anesthesia, average norepinephrine infusion rate, stable period alpha and theta band power, PSI, and hypnotic drug concentration (either MAC or TCI).

To allocate scores to each variable, a decision tree algorithm (implemented via the "rpart" package in R) was initially utilized to determine optimal cutoffs concerning specific outcomes, such as favorable versus poor prognosis. When the decision tree did not yield clear splits, a quartile-based distribution scoring system was used. All variables were graded on a 0-3 scale, with higher scores denoting a worse prognosis. Further nuances of the tree design are elucidated below.

To comprehensively assess and score patients, a novel scoring system was created: the $CME_{UA}$ score. The formulation of this score involved various criteria and was rooted in robust statistical techniques.

*General Framework:* For each patient, individual components of the dataset were scored on a scale of 0 to 3. The final $CME_{UA}$ score was computed by aggregating these component scores.

*Score Assignment:* the approach to assigning scores involved two primary strategies:

- Decision Tree-based Cutoffs: Initially, decision tree algorithms were employed to ascertain optimal cutoffs for each variable. The decision tree was constructed by iteratively splitting the data based on the variable value. The splits were determined using the Gini impurity criterion. The complexity parameter was based on the cross validation data to optimize the pruning process and avoid overfitting. The specific cutoffs were determined by the splits made at each decision node and were used to classify the observations into the respective outcome groups.
- Quartile-based Scoring: For variables where the decision tree method did not yield distinct or clinically meaningful splits, we resorted to the quartile distribution method. Here, variables were categorized into quartiles, and scores were assigned either in an ascending or descending manner, contingent on the clinical significance of the variable.

*Specifics of Scoring:*

- PP Variation (RMSE): Derived from the decision tree algorithm, the cutoffs [36.86085, 39.67269, 43.60605] were employed.
- PAM Variation (RMSE): We utilized cutoffs of [12, 23, 26] determined by the decision tree.
- Norepinephrine AUC: Applied decision tree cutoffs of [2.2, 4, 4.5] after logarithmic transformations.
- Lee Score (Lee et al, Circulation. 1999 Sep 7;100(10):1043-9): Used directly from the dataset. It typically includes the following factors: High-Risk Surgery, History of Ischemic Heart Disease, History of Congestive Heart Failure, History of Cerebrovascular Disease, Preoperative Treatment with Insulin, Preoperative Serum Creatinine >2.0 mg/dL. Each factor is assigned one point. The total score correlates with the risk of experiencing major cardiac complications post-surgery. A higher total score indicates a greater risk (0 Points: Low risk, 1 Point: Intermediate risk, 2 or More Points: High risk)
- Alpha and Theta Values: After logarithmic transformations, decision tree-derived cutoffs were used for score assignment.
- PSI: When decision tree-based scoring was not conclusive, quartile distribution was employed.
- Age: Scored using quartile distribution after an inconclusive decision tree analysis.
- PP Baseline: Utilized decision tree cutoffs of [64.16, 82.40, 97.87459].
- TCI and MAC scaled: Data were initially standardized within drug groups, followed by score assignment based on decision tree-derived cutoffs.

*Outcome measures for CMEUA and criteria:* poor prognosis was defined as a composite of fatal outcome, cardiovascular event or cognitive decline. Fatal outcome was sourced from the French INSEE death database. Cardiovascular outcomes included stroke and myocardial infarction. Cognitive decline was measured before surgery, at hospital discharge, and 2.5 years later using MoCA and T-MoCA (adjusted to a 22-point scale) test. Scores were then normalized and a drop of one standard deviation from baseline indicated cognitive decline. A favorable prognosis defined as no fatal, cardiovascular, or cognitive decline outcomes at the 2.5-year. Cognitive Decline Outcome Assessment: Cognitive assessments were performed at entry to the study, typically the day before surgery, at discharge from the hospital or on Day 3 (whichever was earlier), and 2.5 years post-surgery. The use of both MoCA and T-MoCA tools across these three timepoints allowed for a comprehensive assessment of patients' cognitive changes over time and post-surgery. The MoCA is a cognitive screening tool that takes approximately 15 minutes to administer. It evaluates multiple cognitive domains: attention and concentration, executive functions, memory, language, visuo-constructional skills, conceptual thinking, calculations, and orientation. The T-MoCA is a telephone version of the MoCA and omits the visuospatial/executive and naming component of the original MoCA, resulting in a maximum score of 22. Consistency across assessments was ensured by converting MoCA scores to a 22-point scale, by excluding the visuospatial/executive and naming component values. This was done to facilitate direct comparison with the T-MoCA scores. The use of T-MoCA was prioritized for long-term follow-up to minimize loss-to-follow-up rates. To identify cognitive decline, we calculated z-scores as follows:

Z-score = (patient baseline score - mean baseline score from controls) / (standard deviation (SD) from control group)

To account for the learning effect at 2.5 years, one of the three versions of the T-MoCA that hadn't been previously used for each patient was carefully selected. Large negative z-scores showed deterioration in cognitive function from baseline. Ultimately, cognitive decline at 2.5 years was defined by the presence of a subjective cognitive complaint together with a z-score at 2.5 years ≤ -1 SD.

Statistical Analysis: the primary endpoint was to ascertain a significant association between the CMEUA score and poor prognosis. The score's association with the outcome was evaluated using both Odds Ratio and F1-score metrics.

The secondary endpoint focused on gauging the discriminatory capability of the CMEUA for the composite outcome. To this end, the Receiver Operator Curve (ROC) and the Area Under the Curve (AUC) metrics were ysed. To evaluate the predictive accuracy of our metrics, the dataset was divided into a training set (70%) and a test set (30%) using a random seed of 123 to ensure reproducibility. Furthermore, the ROC-AUC of the CMEUA score was juxtaposed against a clinical model that incorporated age, the ASA score, the existence of hypertension and the Lee Score. Differences between the AUCs were tested for statistical significance using the DeLong test. Finally, the ROC-AUC (AUROC) of the CMEUA score was studied for each level of the composite score using multinomial analysis.

To further validate the score, sensitivity analyses were conducted. Employing a cross-validation methodology, it was opted to use for k-fold validation. To gauge the inherent uncertainty and robustness of the CMEUA model, 2000 bootstrap iterations were undertaken, particularly evaluating the F1 score's stability.

For power analysis, it was aimed for 80% power with an alpha level of 0.05. An odds ratio of 2 was anticipated and a baseline probability of 40% was set for the logistic regression. It was accounted for a 0.5 cross-correlation correction for potential confounder analysis and projected a 10% loss to follow-up, leading to 150 patients. The significance level was set at 0.05. Categorical variables are presented as counts (%), while continuous variables are expressed as either mean (SD) or median [IQR], contingent on their distribution. For comparative analyses, the $\chi^2$, Fisher's exact test, Student's t-test, or the Mann-Whitney U test were employed, as appropriate. Correlations were evaluated using either Pearson or Spearman correlation coefficients. p-values were adjusted for comparisons using the false discovery rate method. Missing data was addressed by employing the multiple imputation (MICE) method, as explained below. All our analyses were conducted using R software, and the reporting is consistent with the STROBE guidelines.

## Example 2. Results

### Study Population

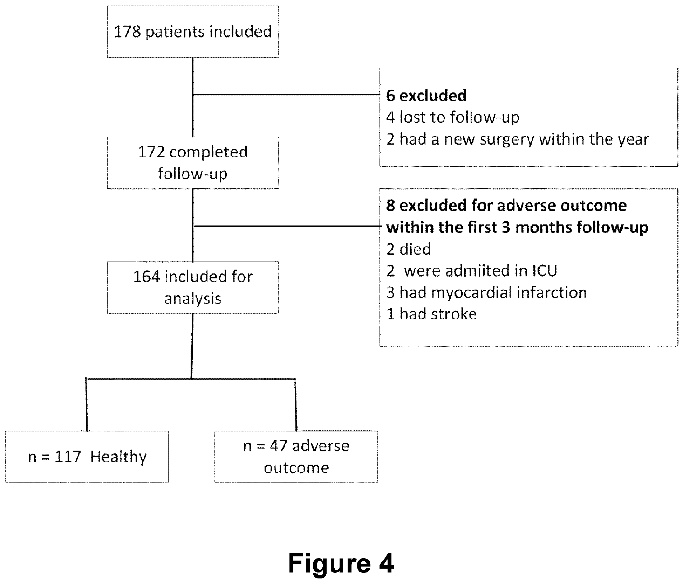

178 patients were enrolled during the study period. 172 completed the 2 years follow up (2 had surgery within the year, 4 were lost to follow-up). Of the 172 patients, 8 patients were excluded from analysis as they presented adverse outcome within the 90 days postoperatively (2 died, 3 had myocardial infarctions, 2 were admitted in ICU and 1 had a stroke (see flowchart in Figure 4). The study analysis comprised of 164 individuals (Age median[IQR]=64[50-74], 68.3% women), the majority (71.3% or n=117) were classified as favorable prognosis, while the remaining 28.7% (n=47) experienced poor prognosis (see table 2). The common poor prognosis detail is shown Table 3.

### General characteristics by outcome

| Characteristics | Overall n=164 | Favorable Prognosis n = 117 | Poor Prognosis n = 47 | p-value |
|---|---|---|---|---|
| Age -yr (median (IQR)) | 64.0 (50.0-74.0) | 60.0 (43.0-71.0) | 74.0 (66.0-79.0) | **<0.001** |
| Sexe, women - n (%) | 112 (68.3%) | 83 (70.9%) | 29 (61.7%) | 0.335 |
| Body Mass Index - kg/m2 (median (IQR)) | 26.4 (23.0-31.2) | 26.4 (23.0-30.9) | 26.4 (23.0-31.9) | 0.933 |
| **Comorbidities - n (%)** | | | | |
| Hypertension | 81 (49.4%) | 43 (36.8%) | 38 (80.9%) | **<0.001** |
| Diabetes | 23 (14.0%) | 13 (11.1%) | 10 (21.3%) | 0.148 |
| Dyslipidemia | 46 (28.0%) | 23 (19.7%) | 23 (48.9%) | **<0.001** |
| Cardiopathy | 37 (22.6%) | 16 (13.7%) | 21 (44.7%) | **<0.001** |

**General characteristics by outcome**

| | | | | |
|---|---|---|---|---|
| Chronic kidney disease | 10 (6.1%) | 4 (3.4%) | 6 (12.8%) | 0.057 |
| Stroke | 13 (7.9%) | 7 (6.0%) | 6 (12.8%) | 0.257 |
| Depression | 16 (9.8%) | 11 (9.4%) | 5 (10.6%) | >0.999 |
| Sleep apnea | 15 (9.1%) | 10 (8.5%) | 5 (10.6%) | 0.904 |
| **Medication** - n (%) | | | | |
| Treated hypertension | 75 (45.7%) | 39 (33.3%) | 36 (76.6%) | **<0.001** |
| Calcium channel inhibiteur | 37 (22.6%) | 19 (16.2%) | 18 (38.3%) | **0.004** |
| Beta-blokers | 41 (25.0%) | 19 (16.2%) | 22 (46.8%) | **<0.001** |
| RAAS inhibitors ☿ | 62 (37.8%) | 33 (28.2%) | 29 (61.7%) | **<0.001** |
| Neuroleptic | 4 (2.4%) | 2 (1.7%) | 2 (4.3%) | 0.692 |
| Benzodiazepine | 31 (18.9%) | 19 (16.2%) | 12 (25.5%) | 0.249 |
| Serotonin recapture inhibitor | 22 (13.4%) | 14 (12.0%) | 8 (17.0%) | 0.545 |
| **ASA Score** - n (%) * | | | | 0.002 |
| 1 | 16 (9.8%) | 14 (12.0%) | 2 (4.3%) | |
| 2 | 105 (64.0%) | 81 (69.2%) | 24 (51.1%) | |
| 3 | 43 (26.2%) | 22 (18.8%) | 21 (44.7%) | |
| Smoker - n (%) | 39 (23.8%) | 32 (27.4%) | 7 (14.9%) | 0.136 |

**General characteristics by outcome**

**Table 2.** General characteristics by outcome

| | | | | |
|---|---|---|---|---|
| Alcohol consumption - n (%) | 14 (8.5%) | 10 (8.5%) | 4 (8.5%) | >0.999 |
| Clinical Frailty Score (median (IQR)) ☽ | 3.0 (3.0-4.0) | 3.0 (2.0-4.0) | 4.0 (3.0-5.0) | **<0.001** |
| **IADL Scale n - (%) +** | | | | **0.003** |
| 1 | 2 (1.2%) | 0 (0.0%) | 2 (4.3%) | |
| 2 | 2 (1.2%) | 0 (0.0%) | 2 (4.3%) | |
| 3 | 20 (12.2%) | 11 (9.4%) | 9 (19.1%) | |
| 4 | 140 (85.4%) | 106 (90.6%) | 34 (72.3%) | |
| **Vision or hearing impairment - n (%)** | 7 (4.3%) | 3 (2.6%) | 4 (8.5%) | 0.202 |

★American Society of Anesthesiologists, higher ASA scores indicate significant preoperative morbidity

☿ Renin Angiotensin-Aldosterone inhibitors

☽ Scores on Clinical Frailty Scale range from 1 to 9 with higher score indicating greater frailty.

+ IADL denotes for instrumental activities of daily living scale range from 0 (complete dependance) to 4 (complete independence),

**Table 3.** Specific outcome

| Characteristics | Overall n=164 | Favorable Prognosis n = 117 | Poor Prognosis n = 47 | p-value |
|---|---|---|---|---|
| Favorable prognosis (%) | 117 (71.3%) | 117 (100.0%) | 0 (0.0%) | |
| Cognitive decline (%) | 20 (12.2%) | 0 (0.0%) | 20 (42.6%) | |
| Deceased (%) | 16 (9.8%) | 0 (0.0%) | 16 (34.0%) | |
| Cardiovascular event (%) | 11 (6.7%) | 0 (0.0%) | 11 (23.4%) | |

(continued)

| Characteristics | Overall n=164 | Favorable Prognosis n = 117 | Poor Prognosis n = 47 | p-value |
|---|---|---|---|---|
| Myocardial infarction (%) | 6 (4.1%) | 0 (0.0%) | 6 (19.4%) | |
| Stroke (%) | 5 (3.4%) | 0 (0.0%) | 5 (16.1%) | |
| Survival time - days (median (IQR)) | 928.0 (904.8-960.2) | 934.0 (909.0-961.0) | 915.0 (875.5-947.5) | 0.004 |

*Preoperative Variables*

Patients with a poor prognosis were significantly older, with a median age of 74 [66-79] years compared to 60 [43-71] years in the favorable prognosis group (p<0.001). The presence of comorbidities such as hypertension, dyslipidemia, and cardiopathy was more prevalent in the poor prognosis group (all p<0.001, as detailed in Table 1). This group also demonstrated higher utilization of antihypertensive medications, including calcium channel inhibitors and beta-blockers (all p<0.001). In contrast, the favorable prognosis group predominantly had ASA scores of 1 and 2 (81.2% vs. 55.4%, p<0.002). Increased frailty, indicated by higher scores on the Clinical Frailty Scale (p<0.001), and lower cognitive performance, as reflected in T-MoCA scores (p=0.004), were associated with poor prognosis. While pain intensity was initially linked to poor prognosis (p=0.021), this association was not significant after adjusting for age.
A dendrogram-based analysis revealed eight clusters of highly correlated variables. To reduce redundancy, one variable from each cluster was selected for its correlation with the outcome. The chosen variables included age, hypertension status, cardiovascular risk factors, Clinical Frailty Scale, baseline T-MoCA scores, history of cardiovascular disease, Lee Score, and dyslipidemia status. After stepwise regression in constructing the Clinical Model, only age, hypertension status, and history of cardiovascular disease remained as significant predictors.

*Intraoperative Variables*

A comparative assessment of intraoperative variables between groups was conducted, accounting for the fact that surgery duration, type, and anesthetic characteristics did not differ significantly between groups (Table 6). Hemodynamic parameters, including mean arterial pressure (MAP), diastolic blood pressure (DBP), and duration of intraoperative hypotension, showed no significant differences between groups. However, pulse pressure (PP) and systolic blood pressure (SBP) were notably higher in the poor prognosis group across reference values, average values, and variability during anesthesia (p-values ranging from 0.021 to <0.001, detailed in Table 4). These differences persisted even after adjusting for hypertension status.
Heart rate (HR) variability was found to be lower in the poor prognosis group (13.9 bpm [9.7-20.1] versus 11.5 bpm [8.3-17.0], p=0.039), but this association was not significant post-adjustment for beta-blocker use. Total norepinephrine dosage was higher in the poor prognosis group (0.1 $\mu$g/kg,[0.0-0.5], versus 0.0 $\mu$g/kg [0.0-0.3], p=0.023).
Stable anesthesia phases revealed lower Propofol Target-Controlled Infusion (TCI) levels in the poor prognosis group (3.2 $\mu$g/ml [2.6-3.9] versus 3.5 $\mu$g/ml [3.4-4.0], p=0.038), with no significant differences in halogenated gas Minimum Alveolar Concentration (MAC) values. Depth of anesthesia, indicated by the Patient State Index (PSI), was significantly lower in the poor prognosis group (28.0, [23.0-32.0] versus 32.0, [27.0-37.0], p=0.006).
EEG variable analysis showed that all band powers were significantly reduced in patients with poor prognosis (p<0.001 for all parameters, see Table 4). Multivariate regression confirmed the significance of these findings, even after adjustments for anesthetic concentration and patient age (p<0.001 for all parameters). Dendrogram-based analysis identified six clusters of highly correlated intraoperative variables (Figure 7). To minimize redundancy, one variable from each cluster was chosen for its correlation with patient outcomes. These included Alpha and Theta band powers, Total power, Hypnotic drug concentrations, PP variability, and Norepinephrine dosage. Subsequent stepwise regression analysis retained Alpha Band Power, PP variability, and Norepinephrine dosage as significant prognostic indicators.

| Hemodynamic parameters | Overall n=164 | Favorable Prognosis n = 117 | Poor Prognosis n = 47 | p-value |
|---|---|---|---|---|
| Percentage of time with PAM < 65 mmHg | 17.4 (5.0-33.3) | 18.8 (5.0-35.0) | 15.4 (2.0-32.2) | 0.553 |
| Percentage of time with PAM < 20% of baseline value | 50.0 (20.0-74.3) | 48.1 (16.0-76.2) | 54.2 (32.3-71.1) | 0.501 |

(continued)

| Hemodynamic parameters | Overall n=164 | Favorable Prognosis n = 117 | Poor Prognosis n = 47 | p-value |
|---|---|---|---|---|
| **Mean Arterial blood Pressure (MAP) - mmHg** | | | | |
| Baseline value of MAP (median (IQR)) | 93.7 (13.0) | 92.7 (12.7) | 96.2 (13.8) | 0.130 |
| Average value of MAP during the procedure (median (IQR)) | 75.7 (70.3-80.7) | 75.3 (69.7-80.2) | 76.3 (73.0-81.7) | 0.239 |
| RMSE of MAP during the procedure (median (IQR)) | 21.2 (13.9-27.3) | 21.1 (13.4-27.7) | 22.1 (16.7-26.7) | 0.310 |
| **Systolic Blood Pressure (SBP) - mmHg (median (IQR))** | | | | |
| Baseline value of SBP (median (IQR)) | 138.8 (20.0) | 134.4 (18.3) | 149.8 (20.1) | **<0.001** |
| Average value of SBP during the procedure (median (IQR)) | 109.2 (102.6-115.6) | 107.6 (102.0-114.2) | 111.6 (105.7-121.5) | **0.021** |
| RMSE of SBP during the procedure (median (IQR)) | 31.8 (19.9-46.5) | 27.7 (18.3-43.4) | 43.5 (29.8-50.4) | <0.001 |
| **Diastolic Blood Pressure (DBP) - mmHg (median (IQR))** | | | | |
| Baseline value of DBP (median (IQR)) | 71.1 (13.5) | 71.8 (12.9) | 69.4 (14.9) | 0.338 |
| Average value of DBP during the procedure (median (IQR)) | 58.3 (53.1-65.1) | 58.6 (52.9-64.8) | 58.0 (53.8-66.2) | 0.830 |
| RMSE of DBP during the procedure (median (IQR)) | 15.7 (10.8-21.3) | 16.7 (11.3-21.8) | 13.0 (9.9-20.3) | 0.116 |
| **Pulsed pressure (PP) - mmHg (median (IQR))** | | | | |
| Baseline value of PP | 64.2 (54.4-82.4) | 60.6 (51.1-72.2) | 83.0 (64.1-93.0) | **<0.001** |
| Average value of PP during the procedure | 50.1 (44.2-56.3) | 49.4 (43.3-54.0) | 53.6 (45.8-59.2) | **0.021** |
| RMSE of PP during the procedure | 17.6 (11.9-32.2) | 15.1 (11.2-24.9) | 36.2 (15.5-41.8) | **<0.001** |
| **Mean Heart Rate (HR) - bpm (median (IQR))** | | | | |
| Baseline value of HR | 68.1 (57.9-79.1) | 69.7 (59.8-79.5) | 65.8 (54.2-77.2) | 0.061 |

Average value of HR during 60.7 (10.3) 61.5 (10.1) 58.6 (10.5) 0.113 the procedure

**Table 4.** Hemodynamic parameters by outcome

| | | | | |
|---|---|---|---|---|
| RMSE of HR during the procedure | | 12.7 (9.2-19.4) | 13.9 (9.7-20.1) | 11.5 (8.3-17.0) | **0.039** |
| **Norepinephrine total dose - $\mu$g/kg (median (IQR))** | 0.0 (0.0-0.4) | 0.0 (0.0-0.3) | 0.1 (0.0-0.5) | **0.023** |

**Table 5.** EEG parameters by outcome

| EEG parameters | Overall n=164 | Favorable Prognosis n = 117 | Poor Prognosis n = 47 | p-value |
|---|---|---|---|---|
| Alpha band power - dB (median (IQR)) | 2979.9 (1349.3-4947.7) | 3906.9 (2772.1-6108.6) | 902.4 (685.6-1374.2) | <0.001 |
| Delta band power - dB (median (IQR)) | 9491.5 (6231.0-18836.6) | 13740.0 (7650.6-24884.1) | 6119.7 (4201.4-7910.4) | <0.001 |
| Theta band power - dB (median (IQR)) | 2243.5 (1240.0-3691.2) | 2562.8 (1750.5-4508.8) | 1027.2 (721.3-1827.2) | <0.001 |
| Low Alpha band power - dB (median (IQR)) | 1393.4 (612.7-2460.5) | 1888.3 (1164.3-2980.7) | 453.4 (335.1-775.7) | <0.001 |

(continued)

| EEG parameters | Overall n=164 | Favorable Prognosis n = 117 | Poor Prognosis n = 47 | p-value |
|---|---|---|---|---|
| High Alpha band power - dB (median (IQR)) | 1204.6 (549.2-2452.1) | 1873.7 (1069.1-3194.8) | 433.1 (300.8-642.3) | <0.001 |
| Beta band power (median (IQR)) | 946.9 (594.4-1526.4) | 1175.0 (858.2-1892.3) | 485.9 (306.4-772.4) | <0.001 |
| Total power - dB (median (IQR)) | 17132.8 (10541.3-30248.9) | 21146.6 (15448.4-36436.4) | 9043.5 (6374.4-11679.9) | <0.001 |
| SEF95 - Hz (median (IQR)) ★ | 13.3 (12.1-14.6) | 13.4 (12.2-14.5) | 13.3 (11.8-14.8) | 0.997 |
| PSI (median (IQR)) ✦ | 30.0 (25.0-36.0) | 32.0 (27.0-37.0) | 28.0 (23.0-32.0) | 0.006 |
| Mean Target Controlled Infusion (TCI)- μg/mL (median (IQR)) | 3.5 (3.0-4.0) | 3.5 (3.4-4.0) | 3.2 (2.6-3.9) | 0.038 |
| Minimum alveolar concentration (MAC) (median (IQR)) | 1.1 (0.9-1.2) | 1.1 (0.9-1.2) | 1.0 (0.8-1.3) | 0.935 |

★Denotes for Spectral Edge Frequency, corresponds to the frequency below which 95% of the signal total power is found. SEF95 in 8-15 Hz ensures that patient sedation is appropriate to generate an alpha rhythm, outside the stable region might be the sign of burst suppression (deep sedation) or beta activity (light sedation) ✦ The Patient State Index™ (PSI) is a quantitative EEG index for assessing the level of consciousness during sedation and general anesthesia. The PSI values range from 0 (suppression of EEG) to 100 (fully awake and alert)

**Table 6.** Surgical characteristics by outcome

| Characteristics | Overall n=164 | Favorable Prognosis n = 117 | Poor Prognosis n = 47 | p-value |
|---|---|---|---|---|
| **Orthopaedic surgery (%)** | 120 (73.2%) | 82 (70.1 %) | 38 (80.9%) | 0.225 |
| **Interventional neuroradiology (%)** | 44 (26.8%) | 35 (29.9%) | 9 (19.1%) | 0.225 |
| **Surgery specification** | | | | 0.093 |
| **Joint replacement (%)** | 99 (60.4%) | 67 (57.3%) | 32 (68.1 %) | 0.104 |
| **Aneurysm (%)** | 26 (15.9%) | 19 (16.2%) | 7 (14.9%) | 0.104 |
| **Sinus stenting (%)** | 18 (11.0%) | 16 (13.7%) | 2 (4.3%) | 0.104 |
| **Spine surgery (%)** | 10 (6.1%) | 5 (4.3%) | 5 (10.6%) | 0.104 |
| **Other orthopaedic surgery (%)** | 11 (6.7%) | 10(8.5%) | 1 (2.1%) | 0.104 |
| **Surgery duration - min (median (IQR))** | 160.0 (120.0-195.0) | 160.0 (120.0-180.0) | 155.0 (120.0-200.0) | 0.736 |
| **Spinal anesthesia (%)** | 3 (1.8%) | 2 (1.7%) | 1 (2.1%) | 1.000 |
| **General anesthesia (%)** | 161 (98.2%) | 115 (98.3%) | 46 (97.9%) | 1.000 |
| **Ketamine - mg (mean (SD))** | 7.71 (11.79) | 6.75 (11.21) | 9.81 (12.86) | 0.117 |
| **Anaesthetic drug (%)** | | | | 0.294 |
| **Desflurane (%)** | 12 (7.3%) | 8 (6.8%) | 4 (8.5%) | 0.439 |
| **Propofol (%)** | 87 (53.0%) | 59 (50.4%) | 28 (59.6%) | 0.439 |
| **Sevoflurane (%)** | 65 (39.6%) | 50 (42.7%) | 15 (31.9%) | 0.439 |

*Primary outcome: discrimination ability of the Augmented Model*

The Augmented Model was constructed as described in Example 1. Following stepwise regression, the model included Age, Cardiovascular History, Alpha Band Power, Total Dose of Norepinephrine, and Pulse Pressure Variation. In the test set, the Augmented Model (function F5) achieved an AUC-ROC of 0.948 [95% CI: 0.890 - 0.998]. In comparison, the Clinical Model, which included only clinical variables, yielded an AUC-ROC of 0.819 [95% CI: 0.708 - 0.917]. A DeLong's test was employed to compare the performance of the two models, revealing a significant difference with a p-value of 0.012 (refer to Figure 2). The 95% confidence interval for the difference in AUC ranged from 0.02 to 0.23.

*Secondary outcome: discrimination ability of the Augmented Model across all levels of outcome*

The performance of the Augmented Model was further evaluated for its ability to predict different levels of the outcome using multinomial ROC-AUC analysis:

- Mortality: For predicting mortality, the $CME_{UA}$ model demonstrated an AUC of 0.869 [95% CI: 0.777 - 0.949].
- Cognitive Decline: When distinguishing cognitive decline from other outcomes, the model achieved an AUC of 0.947 [95% CI: 0.910 - 0.977].
- Cardiovascular Event: For cardiovascular events, the AUC was 0.859 [95% CI: 0.720 - 0.957].

## Example 3. Analysis of the CMUEA score

*Primary outcome : association between the score CMEUA and outcome*

The composite $CME_{UA}$ score was constructed as detailed in the methods section. A marked difference was observed between the groups, with a notably higher score in the poor prognosis group: 16.0 (IQR: 14.0-18.0) versus 9.0 (IQR: 7.0-11.0), p-value < 0.001. These two values can thus be considered as two applicable cutoffs for sorting the patients. When one cutoff is to be used, one can chose 13 as the applicable cutoff (increased risk if $CME_{UA}$ score is higher).

**Table 7.** CME score details by outcome

| Score | Overall n=164 | Favorable Prognosis n = 117 | Poor Prognosis n = 47 | p-value |
|---|---|---|---|---|
| **Baseline Score (during awake stage)** | | | | |
| Age score (median (IQR)) | 1.0 (0.0-2.0) | 1.0 (0.0-2.0) | 2.0 (2.0-3.0) | **<0.001** |
| Simplified Clinical Lee's score (median (IQR)) | 1.0 (0.0-1.0) | 1.0 (0.0-1.0) | 1.0 (1.0-2.0) | **<0.001** |
| Pulsed Pressure (PP) score (median (IQR)) | 0.5 (0.0-1.2) | 0.0 (0.0-1.0) | 2.0 (0.5-2.0) | **<0.001** |
| **Heart Score (all stages)** | | | | |
| MAP variation during anesthesia score (median (IQR)) | 2.0 (0.0-2.0) | 2.0 (0.0-2.0) | 2.0 (0.0-2.0) | 0.397 |
| PP variation during anesthesia score (median (IQR)) | 0.0 (0.0-0.0) | 0.0 (0.0-0.0) | 0.0 (0.0-2.0) | **<0.001** |
| Norepinephrine during anaesthesia score (median (IQR)) | 0.0 (0.0-2.0) | 0.0 (0.0-2.0) | 1.0 (0.0-3.0) | 0.191 |
| **Brain Score (stable anaesthesia stage)** | | | | |
| Theta band power score (median (IQR)) | 1.0 (1.0-2.0) | 1.0 (0.0-2.0) | 2.0 (1.0-3.0) | **<0.001** |
| Alpha power score (median (IQR)) | 1.0 (1.0-1.2) | 1.0 (0.0-1.0) | 2.0 (1.0-2.0) | **<0.001** |
| Patient State Index score (median (IQR)) | 2.0 (1.0-3.0) | 1.0 (0.0-2.0) | 2.0 (1.5-3.0) | **0.003** |
| Sedation score (median (IQR)) (TCI, MAC) | 1.0 (0.0-2.0) | 1.0 (0.0-2.0) | 1.0 (0.0-3.0) | 0.261 |
| **Sum of all score (median (IQR))** | 11.0 (8.0-14.0) | 9.0 (7.0-11.0) | 16.0 (14.0-18.0) | **<0.001** |

Using logistic regression, the association between the CMEUA score and poor prognosis was assessed, including their specific subcategories. For every additional point in the CMEUA score between 11 and 23 points, the risk of poor prognosis escalated significantly with an OR[95CI] of 1.97[1.59 - 2.45] (p<0.001). In the defined subgroups for cognitive decline, cardiovascular (CV) event, and mortality, similar ORs of 1.96[1.49 - 2.59], 1.75[1.35 - 2.26], and 1.82 [1.39 - 2.37], respectively were observed-all statistically significant with p-values < 0.001.

To validate these findings, a 10-fold cross-validation methodology was employed. Consistently across the folds, each unit elevation in the CMEUA score nearly doubled the odds of a negative outcome (OR = 1.986, 95% CI: 1.58 to 2.50). Furthermore, the model had an F1 metric of 0.84. A bootstrap analysis with 2000 iterations returned an average F1-score of 0.83. The standard deviation of this metric was 0.044, and its 95% confidence interval spanned from 0.74 to 0.91.

*Secondary outcome : discrimination ability of score CMEUA*

A logistic regression model was built using the training set, considering the CMEUA total score as the sole predictor for the outcome. The performance of this model on the test set yielded an AUC-ROC of 0.911 with 95% CI [0.813 - 0.911].

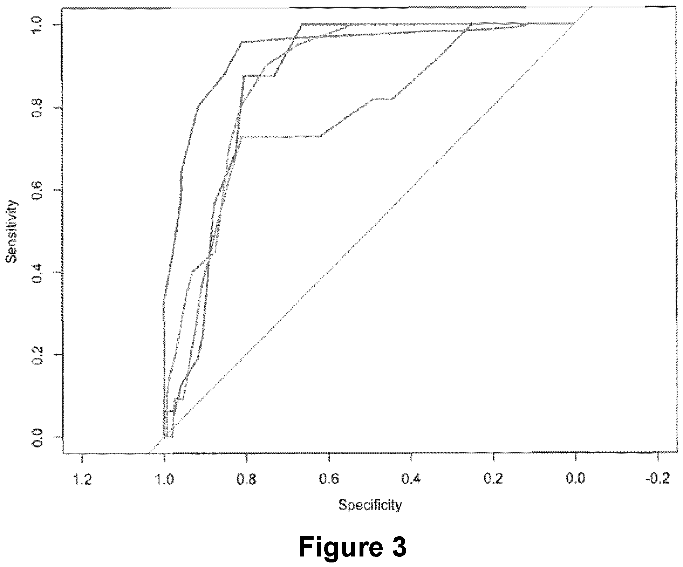

Another logistic regression model was developed using other clinical predictors: age, ASA score (assessing the fitness of patients before surgery), Lee's score, hypertension and cardiopathy condition. The AUC-ROC for this model on the test set was 0.724 with 95% CI [0.588 - 0.724]. To statistically assess the difference in the performance between the two models, DeLong's test was utilized. The test indicated that the AUCs of the two models were significantly different (p = 0.033). The difference in AUC ranged from 0.016 to 0.358 in the 95% confidence interval. To assess the predictive potential of the CMEUA score for different outcome classes, the ROC-AUC values was evaluated for each class (See Figure 3) using a multinomial analysis:

- Favorable prognosis The ability of the score to differentiate between favorable prognosis and the rest yielded an AUC of 0.935 [95% CI: 0.893 - 0.935].
- Mortality: For predicting mortality, the CMEUA score demonstrated an AUC of 0.863 [95% CI: 0.804 - 0.863].
- Cognitive Decline: When distinguishing cognitive decline from other outcomes, the score achieved an AUC of 0.874 [95% CI: 0.815 - 0.874].
- Cardiovascular Event: For cardiovascular events, the AUC was 0.769 [95% CI: 0.622 - 0.769].

**Example 4. Discussion**

The enhanced predictive accuracy of the Augmented Model, as demonstrated in Example 2, firmly supports the integration of intraoperative variables with clinical data for long-term prognosis. The model's considerable improvement in AUC-ROC (AUROC) values compared to the Clinical Model highlights the substantial value added by intraoperative monitoring, particularly hemodynamic and EEG data, to the prognostic process.

The association between intraoperative variables such as Alpha Band Power, PP variability, and Norepinephrine dosage with patient outcomes is striking. Alpha Band Power, in particular, provides a real-time insight into brain function and is a distinguishing characteristic of patients with poor prognosis, an observation that remained significant even after adjusting for age and hypnotic drug concentration. These results position intraoperative EEG not only as a monitor of immediate brain function but also as a predictor of long-term outcomes, including mortality, which is a novel and valuable extension of its conventional use.

In the same vein, PP variability and Norepinephrine dosage reflect the patient's cardiovascular response to the stress of anesthesia and surgery, and the results suggest that PP is a relevant indicator of cardiovascular frailty.

The clinical significance of the above findings is clear: intraoperative data should be a staple in the comprehensive assessment of patient risk. The Augmented Model underscores the importance of tailored cardiovascular and vasopressor management, highlighting the impact of pre-existing cardiovascular conditions on outcomes.

While the potential for preventative interventions based on the model is promising, it could raise ethical concerns and further validation through clinical trials would be of interest, with such trials focusing on controlling cardiovascular and neurocognitive risk factors in line with risk stratification, a strategy supported by existing consensus on the benefits of cardiovascular risk factor management [Piepoli et al. Eur Heart J. 2016;37:2315-2381] and the emerging evidence on early interventions in neurodegenerative diseases [Dubois et al. J Alzheimers Assoc. 2016;12:292-323].

Another score, the CME$_{UA}$ score meticulously amalgamates patient hemodynamics, brain responses under anaesthesia, and pharmacological dynamics into a single predictive framework. This score not only encapsulates diverse parameters but also discriminates patients who are at an elevated risk of poor prognosis in the long term. The development and validation of the CME$_{UA}$ score also mark a paradigm shift in the field, offering clinicians a new, comprehensive tool to prognosticate long term outcomes based on intra-operative data.

The study shows that it is interesting to use baseline PP and its variations, showing its predictive value for mortality, cardiovascular events, and cognitive decline.

The CME$_{UA}$ score highlights the need for tailored cardiovascular and vasopressor management, emphasizing the role of initial cardiovascular conditions on outcomes. It also underscores the importance of individualized depth of anesthesia, with EEG-derived parameters, like lower Alpha band power, as potential outcome indicators.

**Conclusion**

In conclusion, integrating intraoperative variables into patient evaluations could substantially enhance prognostic assessments in the perioperative setting. This integration promises a more dynamic appraisal of the patient's capacity to withstand surgical stress, potentially foreshadowing long-term resilience, or susceptibility to adverse outcomes. Thus, inclusion of intraoperative data as both an immediate surgical management tool and an essential component of extended patient evaluation and screening would be of great interest.

The feasibility of incorporating such data, given the routine nature of intraoperative monitoring, presents a cost-effective opportunity to enrich current screening protocols. This could enable healthcare professionals to identify at-risk patients earlier, facilitating timely and personalized interventions to improve patient outcomes. Future research endeavors should concentrate on standardizing intraoperative data collection methodologies and formulating integrated models for broader clinical implementation.

**Claims**

1. A method for determining whether a subject has an increased risk of having frailty, comprising

   a) Providing data from at least one physiological marker associated with the cerebral state of the subject, and one physiological marker associated with the cardiovascular state of the subject, wherein these data have been measured during anesthesia of the subject
   b) Optionally providing data from at least one pharmacological marker associated with the product administered to the subject during anesthesia
   c) Optionally providing the age of the patient
   d) Calculating numerical values from the data obtained in a) and in b) when provided
   e) Combining the numerical values, optionally normalized, calculated in d) in order to obtain an end value.

2. The method of claim 1, wherein the markers are selected from the groups consisting of

   a. Physiological markers

      i. Markers of cerebral state: alpha wave data, beta wave data, theta wave data, delta wave data, bispectral Index (BIS), Patient State Index (PSI), and Spectral Entropy index (SE),
      ii. Markers of cardiovascular state: mean Arterial Pressure, (mAP), Pulse Pressure (PP) systolic blood pressure (SBP), diastolic blood pressure (DBP), heart rate (HR), Perfusion Index (PI), and Pleth Variability Index (PVI),
      iii. Other markers: Lee's simplified score, cardiovascular history

   b. Pharmacological markers: amount of anesthetic drug, Target Controlled Infusion (TCI), Minimum Alveolar Concentration (MAC), amount of vasopressor, and amount of antalgic drug.

3. The method of claim 1 or 2, wherein the markers comprise alpha wave and Pulse Pressure data.

4. The method of any of claims 1 to 3, wherein the calculation in d) comprises

   a. Calculating the power of the brain wave when brain wave data is used, using the values of the BIS, PSI and SE when such data is used, and /or
   b. Calculating the Root Mean Square Error (RMSE) of the series of values of the mAP, PP, SBP, DPB, HR, PI, PVI as compared to the value before anesthesia, when these markers are used, and/or
   c. Adjusting the amount of anesthetic, vasopressor or antalgic drug according to the time in the operating room and the weight of the subject, when these markers are used, standardizing the TCI or MAC by scaling them to a range between 0 and 1 when these markers are used, and/or
   d. Assigning a discrete score to each marker according to its value and to specific thresholds.

5. The method of any one of claims 1 to 4, wherein the combination is performed through a logistic regression function, which generates an end value, which can be compared to one or more reference values.

6. The method of any one of claims 1 to 4, wherein the function is selected from

a.

$$F1 = a1 - a2 *log(alpha1) + a3*(rmse\_pp),$$

with

- $19.5 \leq a1 \leq 22.5$, preferably $20.5 \leq a1 \leq 21.5$
- $2.5 \leq a2 \leq 3.5$, preferably $2.75 \leq a2 \leq 3.25$
- $0.04 \leq a3 \leq 0.06$

for detecting cardiovascular frailty

b.

$$F2 = b1 + b2*(age) - b3*log(alpha) + b4*(rmse\_pp) + b5*log(dose\_nor\_total),$$

with

- $16.5 \leq b1 \leq 19.5$, preferably $17.5 \leq b1 \leq 18.5$
- $0.060 \leq b2 \leq 077$, preferably $0.065 \leq b2 \leq 0.072$
- $3.10 \leq b3 \leq 3.25$, preferably $3.15 \leq b3 \leq 3.20$
- $0.060 \leq b4 \leq 0.070$, preferably $0.62 \leq b4 \leq 0.067$
- $0.20 \leq b5 \leq 0.35$, preferably $0.25 \leq b5 \leq 0.31$ for detecting neurological frailty

c.

$$F3 = c1 - c2*log(alpha) + c3*(rmse\_pp) + c4*(age) + c5*log(dose\_nor\_total)$$
$$+ c6*(anesthetic\_concentration),$$

with

- $16.5 \leq c1 \leq 19.5$, preferably $17.5 \leq c1 \leq 18.5$
- $3.10 \leq c2 \leq 3.25$, preferably $3.15 \leq c2 \leq 3.20$
- $0.060 \leq c3 \leq 0.070$, preferably $0.62 \leq c3 \leq 0.067$
- $0.060 \leq c4 \leq 0.077$, preferably $0.065 \leq c4 \leq 0.072$
- $0.30 \leq c5 \leq 0.40$, preferably $0.32 \leq c5 \leq 0.38$

for detecting systemic frailty

d.

$$F4 = -d1 + d2×(score\_alpha) + d3×(score\_pp\_var) + d4×(score\_age) +$$
$$d5×(score\_nor\_total) + d6×(score\_anesthetic\_concentration),$$

with

- $6 \leq d1 \leq 8$, preferably $6.5 \leq d1 \leq 7.5$
- $2.4 \leq d2 \leq 3.0$, preferably $2.5 \leq d2 \leq 2.9$
- $0.50 \leq d3 \leq 0.70$, preferably $0.55 \leq d3 \leq 0.65$
- $0.9 \leq d4 \leq 1.1$, preferably $0.95 \leq d4 \leq 1.05$
- $0.35 \leq d5 \leq 0.50$, preferably $0.40 \leq d5 \leq 0.45$
- $0.025 \leq d6 \leq 0.035$, preferably $0.029 \leq d6 \leq 0.033$

for detecting systemic frailty, wherein a [0-3] score has been applied to each marker according to the thresholds of Table 1,and

e.

$$F5 = e1 - e2*log(alpha) + e3*(rmse\_pp) + e4*log(dose\_nor\_total) + e5*(cv\_history\_pre) + e6*(age),$$

with

- $16.5 \leq e1 \leq 19.5$, preferably $18.0 \leq e1 \leq 18.5$
- $3.05 \leq e2 \leq 3.25$, preferably $3.10 \leq e2 \leq 3.20$
- $0.050 \leq e3 \leq 0.060$, preferably $0.52 \leq e3 \leq 0.057$
- $0.25 \leq e4 \leq 0.35$, preferably $0.27 \leq e4 \leq 0.32$
- $1.10 \leq e5 \leq 1.20$, preferably $1.12 \leq e5 \leq 1.16$
- $0.06 \leq e6 \leq 0.07$, preferably $0.06 \leq e6 \leq 0.065$

for detecting systemic frailty.

7. The method of claim 10, wherein the function is selected from

a.

$$F1 = 20.906 - 3.054*log(alpha)+0.052*(rmse\_pp)$$

b.

$$F2 = 18.237 + 0.0686*(age) - 3.184*log(alpha) + 0.0646*(rmse\_pp) + 0.286*log(dose\_nor\_total)$$

c.

$$F3 = 18.0269 - 3.1827*log(alpha) + 0.0655*(rmse\_pp) + 0.0679*(age) + 0.3218*log(dose\_nor\_total) + 0.3543*(anesthetic\_concentration)$$

d.

$$F4 = -6.933 + 2.702\times(score\_alpha) + 0.617\times(score\_pp\_var) + 0.997\times(score\_age) + 0.417\times(score\_nor\_total) + 0.0313\times(score\_anesthetic\_concentration)$$

With

log(alpha) = log value of alpha power (alpha, that has been transformed to rmse_pp = RMSE of the Pulse Pressure
log(dose_nor_total) = log value of the total dose of noradrenaline provided to the patient (pondered by the time in the block and the subject's weight) anesthetic_concentration = standardized TCI or MAC
score(data) = 0, 1, 2, or 3 according to the value of the data and the thresholds, and

e.

$$F5 = 18.256 - 3.159xlog(alpha) + 0.0547x(rmse\_pp) + 0.295xlog(dose\_nor\_total) + 1.144x(cv\_history\_pre) + 0.0637x(age).$$

8. The method of any one of claims 1 to 4, wherein the combination is performed by assigning a discrete score to each marker according to its value and to specific thresholds and summing the discrete scores.

9. The method of any one of claims 1 to 8, comprising comparing the end value to a first predetermined value, wherein the subject has an increased risk of having a general frailty when the end value to the first predetermined value, and/or comparing the end value to a second predetermined value, wherein the subject has a decreased risk of having a general frailty when the end value to the second predetermined value.

10. The method of any one of claims 1 to 9, which is computer implemented.

11. The method of any one of claims 1 to 10, comprising:

   a. receiving, by a processing device, data associated with the markers obtained in relation with the anesthesia of the subject as defined in a) and b) of claim 1, and optionally the age of the subject,
   b. combining said values, by the processing device, in order to generate an output indicative of the risk of general frailty.

12. The method of claim 11, wherein the values used in are sent to a remote server for processing by the processing device and wherein the output is sent to a physician.

13. The method of any one of claims 1 to 12, further comprising determining whether the patient has a latent cardiovascular condition, neurological condition, liver condition, renal condition or cancer, preferably wherein

   a. presence of a cardiovascular condition is determined by electrocardiograms, scanner, MRI, echography, or detection of biomarkers associated with cardiovascular diseases,
   b. neurological condition is determined by performing specific cognitive tests aimed at detecting presence of neurological diseases and/or performing clinical testing such as scanners, electroencephalograms, MRI
   c. presence of a liver condition is determined by an ex *vivo* diagnosis method for the presence of liver fibrosis, steatosis, cirrhosis, or by liver ultrasound, liver stiffness measurement or MRI,
   d. presence of a renal condition is determined by assessing the level of creatinine or the renal clearance,
   e. presence of a cancer is determined by scanner, MRI or measurement of cancer biomarkers in the blood, urine, cerebrospinal fluid or genome of the patient.

14. A computer-implemented method for diagnosing whether a subject has an increased frailty risk, comprising

   a. Receiving inputs from a sender, wherein the inputs are the data of markers that were obtained from an anesthesia of the subject, and optionally the information pertaining to the age of the subject
   b. Performing a method of any one of claims 1 to 13, to obtain an output for the combination of the values received in (a)
   c. optionally comparing the output to a reference value and determining the presence of an increased frailty risk
   d. Sending the output to the sender and/or the presence of an increased frailty risk, if determined
   e. Optionally storing the inputs and the output in a database.

15. A computer implemented method for obtaining information as to the presence of an increased frailty risk in a subject, comprising

   a. Sending inputs to a server, wherein the inputs are the data of markers that were obtained from an anesthesia of the subject, and optionally the information pertaining to the age of the subject
   b. Having the remote server combine the values received in (a) in a function, described in any one of claims 1 to 13, and obtain an output, optionally normalized
   c. optionally having the remote server compare the output to a reference value and determining the presence or absence of an increased frailty risk in the subject
   d. Optionally having the remote server store the inputs and the output in a database
   e. receiving the output from the server and/or the information pertaining to the presence or absence of an increased frailty risk (when determined).

**Figure 1**

**Figure 2**

# Figure 3

178 patients included

6 **excluded**
4 lost to follow-up
2 had a new surgery within the year

172 completed
follow-up

8 **excluded for adverse outcome**
**within the first 3 months follow-up**
2 died
2 were admiited in ICU
3 had myocardial infarction
1 had stroke

164 included for
analysis

n = 117 Healthy

n = 47 adverse
outcome

# Figure 4

**Figure 5**

**Figure 6**

**Figure 7**

Europäisches
Patentamt

European
Patent Office

Office européen
des brevets

# EUROPEAN SEARCH REPORT

Application Number

EP 23 30 6981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILLINGHAM M. ET AL: "Association between intraoperative electroencephalographic suppression and postoperative mortality", BRITISH JOURNAL OF ANAESTHESIA, vol. 113, no. 6, 22 May 2014 (2014-05-22), pages 1001-1008, XP093145419, ISSN: 0007-0912, DOI: 10.1093/bja/aeu105 Retrieved from the Internet: URL:https://www.sciencedirect.com/science/article/pii/S0007091217306578/pdfft?md5=e7ab2dead9f91a3f4b34744d4fb1d45f&pid=1-s2.0-S0007091217306578-main.pdf> * sections "Patient population", "Outcome measures and data collection", "Statistics", "Discussion"; tables 1-3 * | 1-15 | INV. A61B5/021 A61B5/024 A61B5/374 G16H20/10 G16H50/20 G16H50/30 ADD. A61B5/20 |
| A | TOUCHARD CYRIL ET AL: "EEG power spectral density under Propofol and its association with burst suppression, a marker of cerebral fragility", CLINICAL NEUROPHYSIOLOGY, vol. 130, no. 8, 28 May 2019 (2019-05-28), pages 1311-1319, XP085726961, ISSN: 1388-2457, DOI: 10.1016/J.CLINPH.2019.05.014 * sections "BP TIVA score: Brain power spectral density under total intra venous anesthesia (TIVA) by Propofol", "Discussion"; table 1 * | 1-15 | TECHNICAL FIELDS SEARCHED (IPC) A61B G16H |
| A | US 2022/246310 A1 (GARCIA PAUL [US] ET AL) 4 August 2022 (2022-08-04) * paragraphs [0066], [0067] * | 1-15 | |

The present search report has been drawn up for all claims

| Place of search | Date of completion of the search | Examiner |
|---|---|---|
| The Hague | 26 March 2024 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 30 6981**

This annex lists the patent family members relating to the patent documents cited in the above-mentioned European search report.
The members are as contained in the European Patent Office EDP file on
The European Patent Office is in no way liable for these particulars which are merely given for the purpose of information.

**26-03-2024**

| Patent document cited in search report | Publication date | Patent family member(s) | Publication date |
|---|---|---|---|
| US 2022246310 A1 | 04-08-2022 | EP 4041067 A1 | 17-08-2022 |
| | | US 2022246310 A1 | 04-08-2022 |
| | | WO 2021072405 A1 | 15-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017037369 A
- WO 2007128518 A
- WO 2019122406 A
- US 20200390347 A
- WO 0216949 A
- WO 2006082522 A
- WO 2006103570 A
- WO 2013079711 A
- WO 2018050804 A
- WO 2019076830 A
- WO 2020021058 A

### Non-patent literature cited in the description

- **SESSLER et al.** *Anesthesiology*, June 2012, vol. 116 (6), 1195-203
- **SESSLER et al.** *Anesthesiology*, January 2019, vol. 130 (1), 72-82
- **SIGL** ; **CHAMOUN**. *J Clin Monit.*, November 1994, vol. 10 (6), 392-404
- **BOWDLE**. *Anesthesiol Clin*, December 2006, vol. 24 (4), 793-822
- **DROVER** ; **ORTEGA**. *Best Pract Res Clin Anaesthesiol.*, March 2006, vol. 20 (1), 121-8
- **VAKKURI et al.** *Acta Anaesthesiologica Scandinavica*, 2004, vol. 48 (2), 145-53
- **RAMPIL**. *Anesth Analg*, 1983, vol. 62, 186-192
- *Anesthesiology*, 1987, vol. 67, 139-142
- **STRUYS et al.** *Anesth. Analg.*, 2016, vol. 122 (1), 56-69
- **SCHNIDER et al.** *Anesthesiology*, 1998, vol. 88, 1170-82
- **SCHNIDER et al.** *Anesthesiology*, 1999, vol. 90, 1502-16
- **EGER et al.** *Anesthesiology*, 1965, vol. 26 (6), 756-63
- **BOKIL et al.** *J Neurosci Methods.*, 2010, vol. 192, 146-151
- **STOREY J R**. *Stat Soc Ser B Stat Methodol.*, 2002, vol. 64, 479-498
- **VON ELM et al.** *PLoS Med.*, 2007, vol. 4, e296
- **LEE et al.** *Circulation*, 07 September 1999, vol. 100 (10), 1043-9
- **PIEPOLI et al.** *Eur Heart J.*, 2016, vol. 37, 2315-2381
- **DUBOIS et al.** *J Alzheimers Assoc.*, 2016, vol. 12, 292-323